# EUROPEAN PATENT APPLICATION

(11) **EP 4 640 634 A1**
(43) Date of publication of application: **29.10.2025**
(21) Application number: 23907117.8
(22) Date of filing: 21.12.2023
(51) Int. Cl.: C01F 17/235, A01N 25/02, A01N 59/00, A01P 1/00, A01P 3/00, A61K 33/244, A61P 31/04, A61P 31/12, B01J 23/10, B01J 35/39, B01J 35/45, B01J 37/04, B01J 37/12, C08K 3/015, C08K 3/22, C08L 101/00, D06M 11/45

(54) **CERIUM OXIDE NANOPARTICLES AND PRODUCTION METHOD THEREOF**

(30) Priority: 23.12.2022 JP 2022206394
(71) Applicant: Toray Industries, Inc., Tokyo 103-8666 (JP)
(72) Inventor: SEKIGUCHI, Shota, Kamakura-shi, Kanagawa 248-8555 (JP); MASUDA, Tomohide, Kamakura-shi, Kanagawa 248-8555 (JP); MOTOSHIROMIZU, Takahiro, Kamakura-shi, Kanagawa 248-8555 (JP); ITO, Masateru, Kamakura-shi, Kanagawa 248-8555 (JP)
(74) Representative: Kador & Partner Part mbB
(86) International application number: PCT/JP2023/045828
(87) International publication number: WO 2024/135757

(57) **Abstract**

The present invention provides a nanoparticle of cerium oxide, and a dispersion, a resin composition, a fibrous material containing the nanoparticle of cerium oxide, which has high oxidation performance, antiviral performance, and antibacterial performance. The present invention relates to a nanoparticle of cerium oxide including a phosphorus compound adsorbed thereto, wherein a molar ratio (P/Ce ratio) of elemental phosphorus to elemental cerium in the XPS measurement is 0.72 or more, a nanoparticle of cerium oxide including a phosphorus compound adsorbed thereto, wherein the molar ratio of Ce⁴⁺ to total Ce⁴⁺ and Ce³⁺ is 0.070 or more and 0.89 or less, and a nanoparticle of cerium oxide, produced by adding an oxidizing agent to a solution containing condensed phosphoric acid or/and a salt thereof and cerium (III) ion. The present invention also relates to a dispersion, resin composition, fibrous material, containing the nanoparticle.

## Description

### Technical Field

The present invention relates to a nanoparticle of cerium oxide, and a dispersion, an oxidizing agent, an antiviral agent, an antibacterial agent, a resin composition, a resin product, a fibrous material, and a fibrous product containing the nanoparticle. The present invention also relates to a method of producing the nanoparticles of cerium oxide.

### Background Art

In recent years, as awareness of safety and hygiene management has increased, antibacterial technology that decomposes the deleterious material and microorganisms has been attracting attention. For example, titanium oxide has an ability of oxidatively decomposing organic materials through its photocatalytic properties, and its performance has been evaluated in the decomposition reaction of organic dyes, etc. In addition to its use as the antibacterial agent, these oxidative decomposition properties are expected to be used to decompose various deleterious materials, for example, small molecules such as acetaldehyde and ammonia, allergens, and viruses.

On the other hand, the nanoparticle of cerium oxide (nanoceria) has catalytic activity similar to that of an oxidizing enzyme such as oxidase or peroxidase, and is expected to be used as the oxidizing agent. Since these catalytic activities do not require a special light source such as ultraviolet light, they are expected to be used to decompose the deleterious materials in situations where it is difficult to use titanium oxide, such as indoors or in the dark.

In the case of using easily aggregated metal nanoparticle as an oxidizing agent, etc., a method is used to stably disperse the nanoparticle by later adding a stabilizer to a dispersion of the nanoparticle, which has been synthesized without adding the stabilizer.

Here, Patent Literature 1 discloses a whitening agent in which cerium oxide particles are dispersed. It is disclosed that addition of the metaphosphoric acid makes the whitening agent less sensitive to pH and improves dispersion stability.

The Patent Literature 2 also discloses the use of the phosphoric acid, phosphonic acid, and their salts as stabilizers to treat metal nanoparticles such as cerium oxide for stable dispersion of particles. In particular, paragraph 0023 of said literature discloses that phosphoric acid is preferably used.

### Citation List

### Patent Literature

Patent Literature 1: Japanese Patent Laid-Open Publication No. 2020-045291
Patent Literature 2: Japanese Translation of PCT International Application Publication No. 2005-500436

### Summary of Invention

### Technical Problem

The Patent Literature 1 discloses nanoparticles, which are obtained by a production method in which the metaphosphoric acid is added as a stabilizer after the formation of nanoparticles of cerium oxide (hereinafter referred to as "post-addition"). In addition, the Patent Literature 2 discloses nanoparticles, which are obtained by a production method in which the phosphoric acid is post-added as a stabilizer. However, the nanoparticles disclosed in Patent Literature 1 or 2 have a lower oxidation performance.

As described above, the nanoparticles of cerium oxide, which are produced by using condensed phosphoric acid containing metaphosphoric acid or phosphoric acid as a stabilizer have a problem that the oxidation performance and the antiviral performance are low.

Therefore, the present inventors further investigated aiming at finding nanoparticles of cerium oxide having high oxidation performance which are obtained by using condensed phosphoric acid as a stabilizer. The present inventors also investigated the antiviral performance and the antibacterial performance of the nanoparticles.

### Solution to Problem

In order to solve the above problem, the present inventors focused their attention on and investigate a method of producing nanoparticle of cerium oxide. As a result, the present inventors have found that nanoparticles of cerium oxide produced by a production method in which an oxidizing agent is added to a solution containing condensed phosphoric acid or/and a salt thereof and cerium (III) ion (hereinafter referred to as "pre-addition") and a dispersion containing the particles have high oxidation performance, antiviral performance and antibacterial performance. The present inventors also have found that processed products such as a resin composition and a fibrous material, which contain such particles, also have high antiviral performance.

The present invention is as follows.
(1) A nanoparticle of cerium oxide including an inorganic acid adsorbed onto its surface, wherein a zeta potential at pH 7 is 0 mV or less.
(2) A nanoparticle of cerium oxide including a phosphorus compound adsorbed thereto, wherein a molar ratio (P/Ce ratio) of elemental phosphorus to elemental cerium in the XPS measurement is 0.72 or more.
(3) The nanoparticle of cerium oxide according to (2), wherein the phosphorus compound is condensed phosphoric acid.
(4) The nanoparticle of cerium oxide according to (2) or (3), wherein the XRD spectrum has peaks in the ranges of 2θ = 30.5° or more and 32.0° or less and 41.5° or more and 43.0° or less.
(5) A nanoparticle of cerium oxide including a phosphorus compound adsorbed thereto, wherein a molar ratio of Ce⁴⁺ to the total Ce⁴⁺ and Ce³⁺ in the XPS measurement is 0.070 or more and 0.89 or less.
(6) The nanoparticle of cerium oxide according to (5), wherein the phosphorus compound is condensed phosphoric acid.
(7) The nanoparticle of cerium oxide according to (5) or (6), wherein the XRD spectrum has peaks in the ranges of 2θ = 30.5° or more and 32.0° or less and 41.5° or more and 43.0° or less.
(8) A nanoparticle of cerium oxide, produced by the following step:
   Step a): a step in which an oxidizing agent is added to a solution containing condensed phosphoric acid or/and a salt thereof and cerium (III) ion.
(9) The nanoparticle of cerium oxide according to (8), wherein the pH of the solution at the time of adding the oxidizing agent is 5 or more.
(10) The nanoparticle of cerium oxide according to (8) or (9), wherein the condensed phosphoric acid is polyphosphoric acid or metaphosphoric acid.
(11) The nanoparticle of cerium oxide according to (10), wherein the polyphosphoric acid is pyrophosphoric acid, tripolyphosphoric acid, tetrapolyphosphoric acid, pentapolyphosphoric acid, or hexapolyphosphoric acid.
(12) The nanoparticle of cerium oxide according to (10), wherein the metaphosphoric acid is trimetaphosphoric acid, tetrametaphosphoric acid, pentametaphosphoric acid, or hexametaphosphoric acid.
(13) The nanoparticle of cerium oxide according to any one of (8) to (12), wherein the nanoparticle of cerium oxide is produced by steps further including:
   Step b): a step in which the solution obtained in the step a) is subjected to hydrothermal treatment.
(14) The nanoparticle of cerium oxide according to any one of (8) to (13), wherein APHA of a dispersion containing 0.1 mass% of the nanoparticle is 400 or less.
(15) The nanoparticle of cerium oxide according to any one of (8) to (14), wherein a zeta potential at pH 7 is - 20 mV or less.
(16) A dispersion including the nanoparticle of cerium oxide according to any one of (1) to (15).
(17) An oxidizing agent including the nanoparticle of cerium oxide according to any one of (1) to (15).
(18) An antiviral agent including the nanoparticle of cerium oxide according to any one of (1) to (15).
(19) An antibacterial agent including the nanoparticle of cerium oxide according to any one of (1) to (15).
(20) A resin composition including the nanoparticle of cerium oxide according to any one of (1) to (15).
(21) A resin product including the resin composition according to (20).
(22) The resin product according to (21), wherein the resin product is selected from the group consisting of an automobile interior material, an electrical appliance casing, a hanging strap, a handrail, a doorknob, a partition board, and a paint.
(23) A fibrous material including the nanoparticle of cerium oxide according to any one of (1) to (15).
(24) A fibrous product including the fibrous material according to (23).
(25) The fibrous product according to (24), wherein the fibrous product is selected from the group consisting of a mask, protective clothing, a filter, a mat, a chair, a gown, a lab coat, a curtain, a sheet, an automobile interior material, and a wipe.
(26) A method of producing a nanoparticle of cerium oxide, including the following step:
   Step a): a step in which an oxidizing agent is added to a solution containing condensed phosphoric acid of the following general formula (I) or/and a salt thereof and cerium (III) ion.
(27) A method of producing the nanoparticle of cerium oxide according to (26), further including:
   Step b): a step in which the solution obtained in the step a) is subjected to hydrothermal treatment.
(28) A method of producing the nanoparticle of cerium oxide according to (26) or (27), wherein APHA of a 0.1 mass% dispersion of the nanoparticle of cerium oxide is 400 or less.

### Advantageous Effects of Invention

The nanoparticle of cerium oxide of the present invention or a dispersion containing the nanoparticle exhibits high oxidation performance, antiviral performance, and antibacterial performance. The nanoparticle of cerium oxide of the present invention, or the dispersion or processed product such as resin composition or fibrous material, containing the nanoparticle, can be used as an oxidizing agent, or an antiviral agent having high performance to inactivate various viruses, and antibacterial agent.

### Description of Embodiments

The nanoparticle of cerium oxide of the present invention may be herein referred to simply as the nanoparticle of the present invention, and the dispersion containing the nanoparticle of cerium oxide of the present invention may be herein referred to simply as the dispersion of the present invention.

The nanoparticle of cerium oxide of the present invention is characterized by the zeta potential at pH 7 of 0 mV or less and including an inorganic acid adsorbed on the surface thereof.

The zeta potential is one of the values that describe the electrical properties at an interface of a colloid in a solution. When charged colloids are dispersed in a solution, an electric double layer is formed on the surface of the colloid by the counter ion to the surface charge of the colloid. The potential on the colloid surface at this time is called the surface potential. The electric double layer is formed by the electrostatic interaction of the surface charges of the colloid. Therefore, the ion is more strongly fixed on the colloid side. Among the electric double layers, the layer in which the counter ions are strongly fixed on the colloid surface by electrostatic interaction is called a fixed layer, and the potential of the fixed layer is called a fixed potential. When the colloid is moved relative to the solution, the fixed layer moves with the colloid. At this time, there is a boundary surface outside of the fixed layer, as viewed from the colloid, that moves with the colloid due to the viscosity of the solution. This is called a slip surface or a shear surface. The potential of this slip surface when the potential at a point far enough from the colloid is zero is defined as the zeta potential. Thus, since the zeta potential changes depending on the surface charge of the colloid and the surface charge changes due to the adsorption and desorption of protons depending on the pH, the present invention is based on the value in a solution at pH 7. In addition, since the distance to the slip surface is generally smaller compared to the size of the colloid, the surface of the colloid can also be approximately expressed as the slip surface.

The zeta potential can be determined by utilizing interfacial electrokinetic phenomena such as electrophoresis, electroosmosis, backflow potential, or precipitation potential, and can be measured by a method such as microscopic electrophoresis, electrophoresis by rotating diffraction grating method, laser Doppler electrophoresis, ultrasonic vibration potential method, or electrokinetic acoustic method. These measurements can be performed using a zeta potential measuring device. The zeta potential measuring devices are commercially available from Otsuka Electronics Co., Ltd., Malvern Instruments Ltd., Rank Brother Ltd., PenKem Inc., and others.

Any of the above devices can be used to measure the zeta potential, but laser Doppler electrophoresis is the most common method. Laser Doppler electrophoresis is a measurement method that utilizes the Doppler effect, in which light or sound waves strike an object moving due to electrophoresis and change their frequency when scattered or reflected.

When measuring the zeta potential, the nanoparticles of the present invention are dispersed in a solution containing an electrolyte, such as a sodium chloride solution or HEPES buffer solution, and the scattered light or reflected light of the dispersed nanoparticle is detected and measured. Nanoparticles of larger size makes it possible to detect the scattered light or reflected light at lower concentrations.

The specific conditions for measuring the zeta potential of the nanoparticle of the present invention using the laser Doppler method are not particularly limited. For example, the nanoparticle is dissolved in HEPES acid buffer (10 mM, pH 7) so that the concentration is 0.1 wt% or more and 1 wt% or less, or 0.001 wt% or more and 1 wt% or less, and this solution is placed in a measurement cell and placed in the zeta potential measurement device based on the laser Doppler electrophoresis method, and the zeta potential can be measured at room temperature. As the zeta potential measuring device, for example, ELS-Z from Otsuka Electronics Co., Ltd., etc. can be utilized.

The nanoparticle including the inorganic acid of the present invention adsorbed onto its surface has a zeta potential at pH 7 of 0 mV or less, preferably -10 mV or less, and more preferably -20 mV or less.

In the present specification, the term "nanoparticle of cerium oxide" is a concept that also encompasses a particle including an inorganic acid or a phosphorus compound, described below, adsorbed on its surface. The inorganic acid is a collective term for acids that do contain no carbon atom. The phosphorus compound is a collective term for compounds containing elemental phosphorus. In the present specification, the inorganic acid mentioned above includes phosphoric acid and condensed phosphoric acid, and the phosphorus compound mentioned above also includes the phosphoric acid and the condensed phosphoric acid. The preferred inorganic acid is the condensed phosphoric acid. The preferred phosphorus compound is the condensed phosphoric acid.

The nanoparticle of the present invention is characterized by including the inorganic acid adsorbed onto its surface as mentioned above.

In the present specification, the condensed phosphoric acid refers to a product of dehydration condensation of two or more phosphoric acid molecules. The polyphosphoric acid refers to the linear condensed phosphoric acid, the cyclic phosphoric acid refers to the cyclic condensed phosphoric acid, and the metaphosphoric acid refers to a mixture of the polyphosphoric acid and the cyclic phosphoric acid. The cyclic phosphoric acid encompasses ultraphosphoric acid having a net-like structure.

Phosphoric acid undergoes dehydration condensation by heating. Upon condensation, phosphoric acid becomes linear polyphosphoric acid, cyclic phosphoric acid, metaphosphoric acid which is a mixture of linear polyphosphoric acid and cyclic phosphoric acid, or ultraphosphoric acid, and finally becomes diphosphorus pentoxide (tetraphosphorus decaoxide). In the present specification, the condensed phosphoric acid includes the condensates of these phosphoric acids, that is, polyphosphoric acid, cyclic phosphoric acid, ultraphosphoric acid, metaphosphoric acid and diphosphorus pentoxide. As a counterion of the condensed phosphoric acid in the condensed phosphate salt, arbitrary ions such as lithium ions, sodium ions, potassium ions, or ammonium ions can be used.

The condensed phosphoric acid preferably used in the present invention is polyphosphoric acid or metaphosphoric acid. As polyphosphoric acid, pyrophosphoric acid, tripolyphosphoric acid, tetrapolyphosphoric acid, pentapolyphosphoric acid, or hexapolyphosphoric acid can be more preferably used. As metaphosphoric acid, trimetaphosphoric acid, tetrametaphosphoric acid, pentametaphosphoric acid, or hexametaphosphoric acid can be more preferably used.

Furthermore, polyphosphoric acids and metaphosphoric acids may be sold as mixtures of those having higher degrees of polymerization or those having different degrees of polymerization, and such mixtures may be used in the present invention.

The nanoparticle of cerium oxide of the present invention is a nanoparticle of cerium oxide including a phosphorus compound adsorbed thereto, which is characterized in that a ratio (P/Ce ratio) of elemental phosphorus to elemental cerium in the XPS measurement is 0.72 or more.

XPS can provide information on elements and valence, and the peak area ratio of the obtained spectrum can be used to quantify the ratio of the elemental cerium to the elemental phosphorus. To calculate the molar ratio (P/Ce ratio) of elemental phosphorus to elemental cerium, the obtained spectrum is subjected to horizontal axis correction so that P2p main peak is 133.0 eV, and then the peak area of the elemental phosphorus is divided by the peak area of the elemental cerium to calculate the P/Ce ratio. All of the nanoparticle of cerium oxide of the present invention has the ratio (P/Ce ratio) of elemental phosphorus to elemental cerium in the XPS measurement being in the range of 0.72 or more. In the nanoparticle of cerium oxide, the ratio (P/Ce ratio) of elemental phosphorus to elemental cerium, obtained by the XPS measurement may be at least 0.72, which is the lower limit at which the oxidase activity, the antiviral activity, antimicrobial activity, etc. are higher, and more preferably at least 0.75, and even more preferably at least 0.80. In the nanoparticle of cerium oxide, the ratio (P/Ce ratio) of elemental phosphorus to elemental cerium obtained by the XPS measurement may be in the range of 0.72 or more and 1,000 or less, which is a range where the oxidase activity, the antiviral activity, antimicrobial activity, etc. are higher, and more particularly 0.72 or more and 100 or less, more preferably 0.75 or more and 10 or less, and even more preferably 0.80 or more and 8.0 or less. When measuring the molar ratio (P/Ce ratio) of elemental phosphorus to elemental cerium by XPS, dry powder of the nanoparticle is used. For example, a dispersion including the nanoparticle of cerium oxide of the present invention which has been subjected to membrane purification, is dried with hot-air at 40°C or more and 120°C or less or lyophilized, and used as a sample. When the nanoparticle is a composite with the film, resins, or fibers, the surface on which the nanoparticle is processed is measured.

The nanoparticle of cerium oxide of the present invention is characterized by having a ratio of Ce⁴⁺ to the total Ce⁴⁺ and Ce³⁺ of 0.070 or more and 0.89 or less in the XPS measurement.

In the nanoparticle of cerium oxide according to the present invention, the molar ratio of cerium (III) to cerium (IV) may be measured by the X-ray photoelectron spectroscopy (XPS). After the horizontal axis correction is performed by the method mentioned above, the peak division of Ce3d is performed to calculate the molar ratio for each Ce ion. Any nanoparticle of cerium oxide of the present invention has a molar ratio of Ce⁴⁺ to the total Ce⁴⁺ and Ce³⁺ obtained by the XPS measurement falling within the range of 0.070 or more and 0.89 or less. In the nanoparticle of cerium oxide, the molar ratio obtained by the XPS measurement may be in the range of 0.070 or more and 0.89 or less, where the oxidase activity, the antiviral activity, the antibacterial activity, and the like become higher, preferably in the range of 0.10 or more and 0.88 or less, and more preferably in the range of 0.20 or mor and 0.86 or less.

When measuring the molar ratio described above by XPS, powder obtained by drying the nanoparticle is used. For example, a dispersion including the nanoparticle of cerium oxide of the present invention which has been subjected to membrane purification, is dried with hot-air at 40°C or more and 120°C or less or lyophilized, and used as a sample. When the nanoparticle is a composite with the film, resins, or fibers, the surface on which the nanoparticle is processed is measured.

The nanoparticle of the present invention is characterized by including a phosphorus compound adsorbed onto its surface as a stabilizer.

The Bragg angle (2θ) of the nanoparticle of cerium oxide according to the present invention may be measured by the X-ray diffraction method (XRD). Information regarding crystallinity can be obtained from the position and intensity of the peak in the obtained XRD spectrum.

The nanoparticle of cerium oxide of the present invention preferably has peaks in the ranges of 2θ = 30.5° or more and 32.0° or less and 41.5° or more and 43.0° or less in the XRD spectrum. These characteristics are detected in the cerium oxide nanoparticles which have undergone hydrothermal treatment among the production methods described below. The nanoparticle of the cerium oxide of the present invention may further include, as peaks derived from the cerium oxide, peaks at the Bragg angle 2θ of 28.5° or more and 29.1° or less, 33.0° or more and 33.2° or less, 47.2° or more and 48.6° or less, 56.1° or more and 56.3° or less, or 59.1° or more and 59.4° or less, in at the XRD spectrum. The diffraction peak is the maximum intensity value in the XRD spectrum. At the time of determining the Bragg angle that indicates the diffraction peak, when the intensity of the obtained spectrum is low, processing such as smoothing may be performed.

In the nanoparticle of cerium oxide having the peaks in the ranges of 2θ of 30.5° or more and 32.0° or less and 41.5° or more and 43.0° or less in the XRD spectrum described above, it is preferable that two peaks are each located in the ranges of the Bragg angle (2θ) of 30.8° or more and 31.8° or less and 41.7° or more and 42.7° or less, respectively, and it is more preferable that the two peaks are each located in the ranges of the Bragg angle (2θ) of 31.1° or more and 31.6° or less and 41.8° or more and 42.3° or less. When measuring the XRD spectrum, powder obtained by drying the nanoparticle is used. For example, a dispersion including the nanoparticle of cerium oxide of the present invention which has been subjected to membrane purification, is dried with hot-air at 40°C or more and 120°C or less or lyophilized, and used as a sample. When the nanoparticle is a composite with the film, resins, or fibers, the surface on which the nanoparticle is processed is measured.

The nanoparticles of cerium oxide of the present invention is produced by adding an oxidizing agent to a solution containing condensed phosphoric acid or/and a salt thereof and cerium (III) ion. That is, the nanoparticle of cerium oxide of the present invention is produced by a production method in which a stabilizer is "pre-added". The synthesis of the nanoparticle of cerium oxide of the present invention is performed using a water-soluble salt of cerium as one of the raw materials, using water or a solvent having a compatibility with water. From the viewpoint of having moderate hydrophilicity and possessing the property of stably dispersing the nanoparticle by forming a complex with a hydroxyl group of a metal oxide, the condensed phosphoric acid or/and a salt thereof is used as a stabilizer, in one embodiment. Uncondensed phosphoric acid is not desirable because it will bind strongly with the cerium ion to form a precipitate.

### <A> Method of Producing Nanoparticle

A dispersion containing the nanoparticles of cerium oxide of the present invention is produced by a production method in which an oxidizing agent is added to a solution containing condensed phosphoric acid or/and a salt thereof and cerium (III) ion (hereinafter referred to as "pre-addition"). Hereinafter, a method of producing a dispersion of nanoparticle of cerium oxide of the present invention will be described.

### [Step a): Step of forming nanoparticle]

The step of forming the nanoparticle of the present invention includes the first step and the second steps described below.

### [Step a -1): First step]

The first step is a step of obtaining a solution containing condensed phosphoric acid or/and a salt thereof and cerium (III) ion. The solution of condensed phosphoric acid or/and a salt thereof used in this step can be prepared by dissolving the condensed phosphoric acid or/and the salt thereof in an arbitrary solvent. The preferred solvent is water or a solvent having compatibility with water. Specific examples of the solvent having compatibility with water include methanol, ethanol, propanol, isopropanol, butanol, tert-butanol, tetrahydrofuran, acetone, dimethylformamide (DMF), dimethyl sulfoxide (DMSO), glycerol, ethylene glycol, propylene glycol, oligoethylene glycol, 2-pyrrolidone, and N-methyl-2-pyrrolidone. When the condensed phosphoric acid or/and a salt thereof is hardly dissolved in a solvent, it may be dissolved by heating or sonication.

The amount of the condensed phosphoric acid or/and a salt thereof may range from 0.01 to 1,000 molar equivalent, preferably from 0.1 to 100 molar equivalent, more preferably from 0.5 to 50 molar equivalent, and most preferably from 1 to 30 molar equivalent, relative to cerium (III) ion.

To obtain a solution containing the condensed phosphoric acid or/and a salt thereof and cerium (III) ion, a solution of the condensed phosphoric acid or/and a salt thereof and a solution containing cerium (III) ion may be separately prepared and mixed. Alternatively, when the solvent for the solution of the condensed phosphoric acid or/and a salt thereof is water or a solvent having a compatibility with water, cerium (III) salt may be added to the solution of the condensed phosphoric acid or/and a salt thereof and mixed, or the condensed phosphoric acid or/and a salt thereof may be added to a solution containing cerium (III) ion and mixed.

A solution containing cerium (III) ion may be prepared by dissolving cerium (III) salt in an arbitrary solvent. Examples of the cerium (III) salts that can be used include inorganic salts such as nitrates, chlorides, sulfates, and the phosphates; organic salts such as acetates and oxalates; cerium complexes having ligands such as NH₃ (ammine) and NO₃ (nitrato); and hydrates. A preferred cerium (III) salt is a nitrate salt. For example, cerium (III) nitrate hexahydrate may be used.

As for the amount of cerium (III) salt, cerium (III) salt may be mixed with a solution of the condensed phosphoric acid and/or a salt thereof so that the final concentration of the reaction liquid ranges from 0.01 mass% to 10 mass%. The mixed solution is preferably mixed for 5 minutes or more until the solution becomes homogeneous.

In the first step, it is preferable that the solution containing the condensed phosphoric acid or/and a salt thereof and cerium (III) ion does not contain trivalent or higher carboxylic acids such as a compound shown below. Even when included, the amount is preferably 0.1 equivalent or less, more preferably 0.01 equivalent or less, relative to cerium (III) ion. Specific examples of the trivalent or higher carboxylic acids include nitrilotriacetic acid (NTA), ethylenediaminetetraacetic acid (EDTA), ethylenediaminodisuccinic acid (EDDS), glycol ether diamine tetraacetic acid (EGTA), diethylenetriamino pentaacetic acid (DTPA), citric acid, hydroxyethyl ethylenediaminetetraacetic acid (HEDTA), polyacrylic acid and/or salts thereof.

When doping the nanoparticle of cerium oxide of the present invention with a metal, a transition metal may also be further added in the first step. The transition metal may be added as a solid metal salt directly to a solution containing the condensed phosphoric acid or/and a salt thereof and cerium (III) ion or cerium (III) salts, or a solution prepared by dissolving the metal salt in an arbitrary solvent may be added to a solution containing the condensed phosphoric acid or/and a salt thereof and cerium (III) ion or cerium (III) salt.

The amount of the transition metal is preferably in the range of 0.0001 moles to 0.3 moles, more preferably in the range of 0.001 to 0.2 moles, per mole of cerium (III) ion. The amount of the transition metal does not include the amount of elements other than the transition metal contained in the transition metal salt.

### [Step a -2): Second step]

The second step is a step of adding an oxidizing agent to the mixed solution obtained in the first step. Examples of the oxidizing agent used in the second step include nitric acid, potassium nitrate, hypochlorous acid, chlorous acid, chloric acid, perchloric acid, halogen, permanganate, chromic acid, dichromic acid, oxalic acid, sulfur dioxide, sodium thiosulfate, sulfuric acid, hydrogen peroxide, oxygen and ozone. Oxygen in the air is sometimes used as the oxidizing agent. The oxidizing agent used in the second step is preferably hypochlorous acid, permanganate, chromic acid, dichromic acid, hydrogen peroxide, oxygen or/and ozone. Among them, hydrogen peroxide is particularly preferred. The amount of the oxidizing agent added may be 0.1 molar equivalents or more and 10 molar equivalents or less, more preferably 0.5 molar equivalents or more and 5 molar equivalents or less, and preferably 0.5 molar equivalents or more and 2 molar equivalents or less, relative to cerium (iii) ion.

When the oxidizing agent is added to a solution containing the condensed phosphoric acid or/and a salt thereof and cerium (III) ion, cerium (III) ion is oxidized to cerium (IV), initiating the formation reaction of the cerium oxide particle, which includes a mixture of Ce₂O₃ and CeO₂. During the reaction, the solution is colored yellow, orange, red, brown, etc. This is coloration caused by the change of cerium (III) ion to cerium (IV), and the degree of coloration is determined by the ratio of cerium (III) to cerium (IV) present on the surface of the nanoparticle of cerium oxide. The end of the reaction can be judged when no color change has occurred.

The formation reaction of the nanoparticle of cerium oxide can be performed at arbitrary pH. However, since the reaction tends to proceed at weakly acidic to basic pH, the pH of the solution at the time of adding the oxidizing agent is preferably pH 5 or more, more preferably adjusted to pH 6 or more, and still more preferably adjusted to pH 7 or more. To adjust the pH, an aqueous solution of sodium hydroxide, an aqueous solution of ammonia, or the like can be used. The pH of the reaction solution may be maintained at 5 or more from the time of addition of the oxidizing agent until the end of the reaction, since the pH of the solution becomes more acidic as the reaction proceeds. The reaction usually completes within 5 minutes to an hour, yielding a dispersion, which contains the nanoparticle of cerium oxide of the present invention. For example, 10 ml of a 10 mass% aqueous solution of cerium (III) nitrate hexahydrate is added to 2.72 g/250 ml of an aqueous solution of sodium polyphosphate (pH 9.3), and then 20 ml of a 1.2 mass% aqueous solution of hydrogen peroxide is added and stirred at room temperature, and the solution turns yellow in about 5 minutes. The particle formation reaction is completed in about 2 hours, and the dispersion of the present invention is obtained.

The reaction of forming the nanoparticle of cerium oxide can be performed at an arbitrary temperature from 4°C to 230°C. When the reaction is performed under cooling, for example, a cool bath such as BBL101 manufactured by Yamato Scientific Co., Ltd. can be used, and when the reaction is performed under heating, for example, a hot bath such as OHB-1100S manufactured by TOKYO RIKAKIKAI CO., LTD. can be used. In either case, the reaction liquid may be placed in a container and cooled, heated, heated to reflux, or hydrothermally treated, while stirring. The material of the container is not particularly limited, and may be glass, plastic, and stainless steel. When the temperature exceeds 100°C, a container capable of withstanding a pressure exceeding 1 atmosphere may be used.

The pH of the dispersion of the present invention may be adjusted after the reaction is completed. The pH of the dispersion of the present invention may range from pH 1 to 12, preferably from pH 2 to 10. The pH may be adjusted by adding a buffer solution, or an acid such as nitric acid, sulfuric acid, or hydrochloric acid, or a base such as ammonia, sodium hydroxide, or potassium hydroxide. In addition, when step b): hydrothermal treatment described below is performed, the pH adjustment of the dispersion may be performed after the step b), or after purification of the dispersion by filtration with ultrafiltration membrane described below, or dialysis with semipermeable membrane.

The dispersion of the present invention can be purified by removing unreacted oxidizing agent and cerium (III) ions and excessive condensed phosphoric acid or/and a salt thereof, remaining in the dispersion after the reaction is completed, by filtration with an ultrafiltration membrane or dialysis with a semipermeable membrane. In this case, the removal is performed so that the concentration of cerium (III) may be 10 mM or less, preferably 5 mM or less. The nanoparticle concentrations can also be increased by filtration with an ultrafiltration membrane, dialysis with a semipermeable membrane. Thereafter, the nanoparticle of cerium oxide may be isolated from the dispersion of the present invention in the manner described below. The aforementioned purification may be performed after the step b): hydrothermal treatment step described below. The condensed phosphoric acid remains adsorbed on the surface of the nanoparticle of cerium oxide obtained after the aforementioned purification.

### [Step b): Hydrothermal treatment step]

In the present invention, the reaction liquid obtained in the step a - 2), after addition of an oxidizing agent, or a dispersion after the above purification or after drying and redispersion, may be subjected to heat treatment at any temperature from 30°C to 230°C. For heating to 100°C or less, it is sufficient to heat the dispersion in a glass container, and for heating to 100°C or more, the hydrothermal treatment is performed in the manner described below. In the present invention, it is preferable to perform the hydrothermal treatment.

In the present invention, the hydrothermal treatment is a step of treatment with water at a temperature higher than 100°C and pressure higher than 101 kPa (1 atm). The hydrothermal treatment can have an effect of improving the colorability of the nanoparticle of cerium oxide. When the dispersion including the nanoparticle of cerium oxide is colored dark orange, red, etc., it is changed to light yellow or light brown by the hydrothermal treatment. The effectiveness of the hydrothermal treatment depends on the temperature and time of the hydrothermal treatment. The higher the processing temperature of the hydrothermal treatment and the longer the reaction time, the greater the improvement for the colorability. The pressure in the hydrothermal treatment can be determined from the saturated water vapor pressure table and the temperature. The hydrothermal treatment may be performed at the temperatures more than 100°C (101 kPa) and 230°C (2.80 MPa) or less, preferably from 105°C (121 kPa) to 200°C (1.55 MPa), and more preferably from 110°C (143 kPa) to 180°C (1.00 MPa). The time can be set arbitrarily between 1 and 180 minutes. Even with the same heat treatment, the colorability is not improved when heated at a temperature not exceeding 100°C or a pressure not exceeding 101 kPa.

In the hydrothermal treatment of the present invention, the reaction liquid after addition of the oxidizing agent obtained in the step a-2) may be placed and heated in a pressure-resistant. For example, the reaction liquid may be placed in a pressure-resistant container, which combines an inner cylindrical container made of PTFE with an outer cylindrical container made of pressure-resistant stainless steel, and heated in an oil bath. Alternatively, the dispersion after purification can be placed in a medium bottle and sterilized using a sterilizer such as LSX-500 manufactured by TOMY

### KOGYO CO.,LTD.

### [Post-treatment of dispersion]

The nanoparticle of cerium oxide of the present invention can be isolated by drying the dispersion of the present invention using an evaporator or a freeze dryer. The nanoparticle of cerium oxide can also be isolated by dropping the dispersion of the present invention onto a glass, plastic, or ceramic substrate and air-drying, drying in a desiccator, or drying with a dryer or a drying machine. The nanoparticle can also be isolated by dropping the dispersion of the present invention onto a heat block and heating to evaporate the solvent. The nanoparticle can also be isolated by drying the dispersion of the present invention with a spray dryer or the like and evaporating the solvent. Alternatively, the nanoparticle can be isolated by centrifuging the dispersion of the present invention to precipitate the nanoparticle of cerium oxide, and removing the supernatant. In addition, the nanoparticle of cerium oxide can also be isolated on the filtration membrane by filtering the dispersion of the present invention using ultrafiltration or suction filtration and completely removing the water. In order to make the drying step in the above operation more efficient, an azeotropic solvent may be added to the dispersion of the present invention, or the solvent in the dispersion may be replaced with a solvent having a lower boiling point. In addition, to make the centrifugation step more efficient, a coprecipitant may be added to the dispersion of the present invention, a solvent may be added to improve the ion strength, or a solvent may be added to reduce the dispersibility of the nanoparticles. Alternatively, prior to the above operation, the dispersion of the present invention may be subjected to ultrafiltration membrane or centrifugation to fractionate the size of the nanoparticles.

The dispersion of the present invention may contain an ion component. Examples of the ion component include, as a component that imparts buffering performance, acetic acid, phthalic acid, succinic acid, carbonic acid, tris(hydroxymethyl)aminomethane (Tris), 2-morpholinoethanesulfonic acid, monohydrate (MES), bis(2-hydroxyethyl)iminotris(hydroxymethyl)methane (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), 2-hydroxy-3-morpholinopropanesulfonic acid (MOPSO), N,N-bis(2-hydroxyethyl)-2-aminoethanesulfonic acid (BES), 3-morpholinopropanesulfonic acid (MOPS), N-tris(hydroxymethyl)methyl-2-aminoethanesulfonic acid (TES), 2-[4-(2-hydroxyethyl)-1-piperazinyl]ethanesulfonic acid (HEPES), 2-hydroxy-N-tris(hydroxymethyl)methyl-3-aminopropanesulfonic (TAPSO), piperazine-1,4-bis(2-hydroxy-3-propanesulfonic acid) (POPSO), 2-hydroxy-3-[4-(2-hydroxyethyl)-1-piperazinyl]propanesulfonic acid (HEPSO), 3-[4-(2-hydroxyethyl)-1-piperazinyl]propanesulfonic acid (HEPPS), (Tricine), N,N-bis(2-hydroxyethyl)glycine (Bicine), and N-tris(hydroxymethyl)methyl-3-aminopropanesulfonic acid (TAPS); and as a component that does not impart buffering performance, sodium chloride and potassium chloride. These ion components can be added to a final concentration of 0.1 mM to 1 M. These ion components may be added to the dispersion after the completion of the reaction in the step a-2), added to the dispersion after the step b) when the hydrothermal treatment of the step b) is performed, added after filtration with the ultrafiltration membrane, or used as a dialysate, and added to the dispersion after dialysis. The ion components may be added to the nanoparticle of cerium oxide to prepare a dispersion.

The dispersion of the present invention may be stored as a dispersion after the reaction is completed, as a purified product filtered through the ultrafiltration membrane or a purified product dialyzed through a semipermeable membrane, from a dispersion after the reaction is completed, or as a nanoparticle of cerium oxide dried and isolated using an evaporator, a spray dryer, a lyophilizer, or the like.

When the dispersion is stored as dry powder, a dispersing agent may be added before or after drying to suppress aggregation of the cerium oxide nanoparticle of the present invention. As the dispersing agent, hydrophilic polymers such as starch, polyvinyl alcohol, polyvinylpyrrolidone, polyacrylic acid, polyethylene oxide, and polyacrylamide, cationic surfactants such as quaternary ammonium salts, anionic surfactants such as higher fatty acid salts and alkylsulfate ester salts, amphoteric surfactants such as alkyl betaines, and non-ionic surfactants such as polyoxyethylene sorbitan fatty acid salts and the polyoxyethylene alkyl ether are preferred; with polyvinyl alcohol, polyvinylpyrrolidone, cationic surfactants, or non-ionic surfactants being more preferred.

In addition, the dispersion of the present invention may be stored as a dispersion containing solvent components such as the above-described azeotropic solvent or ion components added thereto, or it may be stored as a dispersing as a dispersing agent with the pH adjusted. Refrigeration is preferred for storage.

### <B> Characteristics of nanoparticle of cerium oxide

In the present invention, the nanoparticle of cerium oxide includes a mixture of Ce₂O₃ and CeO₂. The cerium oxide may also include hydroxide and oxyhydroxide forms in addition to the oxide forms mentioned above. The above-described oxides may take on a crystalline structure or an amorphous state. The ratio of Ce₂O₃ to CeO₂ can be calculated as the ratio of cerium (III) to cerium (IV), for example, by the X-ray photoelectron spectroscopy (XPS).

The nanoparticle of cerium oxide of the present invention may further include the transition metals of groups 3 to 12 in the periodic table. These metals are expected to improve performance by creating lattice defects when doped on the cerium oxide nanoparticle due to their valence of 2+ to 3+, or by causing a valence change in the cerium oxide due to valence changes such as between 0 and 1+, 1+ and 2+, 2+ and 3+, associated with redox potentials.

These transition metals are more easily doped on the nanoparticle of cerium oxide. From the viewpoint of improving the antimicrobial and antiviral effect, the transition metals of the 4th to 6th period are preferred, and Ti, Mn, Fe, Co, Ni, Cu, Zn, Zr or Ag are more preferred.

These transition metals can be added during synthesis (step a -1)) as organic acid salts such as carboxylate salts and sulfonate salts, phosphorus oxo acid salts such as phosphate salts and phosphonate salts, inorganic acid salts such as nitrate salts, sulfate salts and carbonate salts, as well as halides and hydroxides. They may be any compounds that are dissolved in the synthetic solvent.

When the nanoparticle of cerium oxide of the present invention is a dispersion, the hydrodynamic diameter is analyzed by measuring the dynamic light scattering, deriving the autocorrelation function, analyzing by the Non-Negative Least Squares (NNLS) method, and calculating the cumulant diameter as the particle diameter. For dynamic light scattering measurements, an ELSZ-2000ZS manufactured by Otsuka Electronics Co., Ltd. is used. The hydrodynamic diameter of the nanoparticle of cerium oxide may be 1 to 1,000 nm, preferably 1 to 300 nm, more preferably 1 to 200 nm, still more preferably 1 to 150 nm, and most preferably 1 to 100 nm.

The hydrodynamic diameter is measured with a dispersion containing nanoparticles. The measurements are performed at 25°C, adjusting the particle concentration to 0.001 to 1 mass%, the salt concentration to 100 mM or less, and the pH to 2 to 12. When the particle concentration is low, membrane concentration or evaporation is performed, and when the particle concentration is high, the concentration is adjusted by diluting with a solvent. When the particle concentration of the dispersion is known at the time of measurement, the concentration can be adjusted using the method described above. When the concentration of the cerium oxide is unknown, the concentrations of the cerium ion are determined, for example, by ICP emission spectroscopy (ICP-OES) or ICP mass spectrometry (ICP-MS), and the concentration of cerium oxide is determined assuming that the cerium ions are all CeO₂, and the concentrations are adjusted. When the nanoparticle is the dispersion and does not contain impurities that affect the value of the hydrodynamic diameter, the hydrodynamic diameter may be measured as is. When the dispersion includes the compound which affects the value of the hydrodynamic diameter, other than the nanoparticle which includes cerium oxide, they are removed by the membrane purification, etc. before measurement. When debris is contained in addition to cerium oxide, the debris is removed by centrifugation and the supernatant is measured. The dispersion can also be sonicated before measurement.

The particle diameter of the nanoparticle of the present invention when it is powder can be measured with a granulometer or an electron microscope. The particle diameter of the nanoparticle of the present invention may be 1 to 1,000 nm, and is preferably 1 to 300 nm, more preferably 1 to 200 nm, still more preferably 1 to 150 nm, and most preferably 1 to 100 nm. When the nanoparticle of the present invention forms an aggregate (secondary particles), the particle diameter can be the diameter of the particles themselves (primary particles) that constitute the aggregate, not the diameter of the aggregate. Secondary particles can be observed with a granulometer, and primary particles can be observed with an electron microscope or the like.

The nanoparticle of the present invention denotes the dispersion stability over a broad pH range. In the present specification, a nanoparticle having the dispersion stability means that when the nanoparticle is made into a dispersion, the dispersion does not substantially precipitate when left at room temperature, and specifically, when the nanoparticle is prepared as a dispersion at a concentration of 0.1 mass%, no precipitate is visually observed after centrifugation at 3,000G for 2 minutes. The preferred state for the dispersion stability is a state where no precipitates are visually observed after centrifugation at 6,000G for 2 minutes, and more preferably a state where no precipitates are visually observed after centrifugation at 10,000G for 2 minutes. The nanoparticle of the present invention has the dispersion stability at pH 6 or more and 8 or less, preferably at pH 5 or more and 9 or less, and even more preferably at pH 3 or more and 9 or less.

The nanoparticle of the present invention exhibits the dispersion stability over a wide pH range, and is therefore easily made into products such as a disinfectant as a dispersion. In addition, the dispersion of the nanoparticle is stable, and therefore the dispersion is applied to the resin composition or fibrous material, so that it becomes easy to manufacture the resin composition, fibrous product, and paint compositions containing the nanoparticle.

The reaction liquid, after addition of the oxidizing agent of the present invention, or the dispersion after the above purification or after drying and redispersion, gives a color. The 0.1 mass% dispersion has a value of 400 or more when evaluated by the Hazen color (APHA). APHA is known as an index capable of evaluating unknown coloring substances with high sensitivity.

When the reaction liquid after addition of the oxidizing agent of the present invention is subjected to the hydrothermal treatment, the dispersion which contains the nanoparticle of the present invention becomes transparent or very light yellow, improving the coloration. In the dispersion of the nanoparticle of cerium oxide, which is adjusted to 0.1 mass%, the range of improved coloration may be APHA of 400 or less, preferably APHA of 300 or less, more preferably APHA of 250 or less, and most preferably APHA of 200 or less.

APHA is measured according to the method specified in JIS or using a commercially available measuring device. APHA can be measured using, for example, OME2000 manufactured by Nippon Denshoku Industries Co., Ltd.

APHA is measured with a dispersion containing nanoparticles. The measurements are performed at 25°C, adjusting the particle concentration to 0.1 mass% and the pH to 2 to 12. When the particle concentration is low, membrane concentration or evaporation is performed, and when the particle concentration is high, the concentration is adjusted by diluting with a solvent. When the particle concentration of the dispersion is known at the time of measurement, the concentration can be adjusted using the method described above. When the concentration of the cerium oxide is unknown, the concentrations of the cerium ion are determined, for example, by ICP emission spectroscopy (ICP-OES) or ICP mass spectrometry (ICP-MS), and the concentration of cerium oxide is determined assuming that the cerium ions are all CeO₂, and the concentrations are adjusted. When the nanoparticle is a dispersion and does not contain impurities that affect the APHA value, the APHA of only the solvent (e.g., water) that constitutes the dispersion may be measured as a reference, and then the APHA of the dispersion may be measured. When the dispersion includes a compound which affects the APHA value, other than the nanoparticle which includes cerium oxide, they may be removed by the membrane purification, etc. before measurement. When it is difficult to remove the compound which affects the APHA value, a solution containing the compound may be measured as a reference, and APHA may be measured from the difference. When debris is contained in addition to cerium oxide, the debris can be removed by centrifugation and the supernatant may be measured. The dispersion can also be sonicated before measurement.

When the nanoparticle is a powder, it is redispersed in a solvent before measurement. The solvent is selected from hexane, ethyl acetate, chloroform, methanol, ethanol, DMSO, water, or a mixed solvent thereof. Of these, water is preferred. However, the pH can be adjusted to increase solubility, or a solvent mixture of water and an organic solvent having compatibility with water such as methanol, ethanol, or DMSO can be used. When a mixed solvent is used, the mixing ratio may be water : organic solvent = 1:99 to 99:1. When the solubility of the nanoparticles in polar solvents is low, hexane, ethyl acetate or chloroform can also be used. To increase solubility, heating, cooling, or ultrasonication may be performed.

The nanoparticles of cerium oxide of the present invention or a dispersion thereof may be sterilized before use. Examples of the sterilization methods include a method of passing through a sterilization filter, a method by autoclaving (e.g., at 120°C for 20 minutes), and a method of irradiation with 254 nm ultraviolet light.

### <C> Performance and usage of nanoparticle of cerium oxide of the present invention

The nanoparticle of cerium oxide of the present invention or a dispersion thereof has a high oxidation performance. Therefore, the nanoparticle of cerium oxide of the present invention can be used as the oxidizing agent. In the present specification, an "oxidizing agent" includes an "oxidation catalyst". In the present specification, the "oxidation performance" includes both the performance of stoichiometric amounts of the cerium (IV) in the nanoparticle to oxidize the target substance and the performance of the nanoparticle to oxidize the target substance by acting as an oxidation catalyst (oxidation catalytic performance). The oxidation performance can be evaluated by a method of the measuring the oxidase activity described below, a method of measuring the peroxidase activity, or the like. The oxidase activity can be determined by the method shown in A. Asati, Angew. Chem. Int. Ed. 2009, 48, 2308-2312. Specifically, a dilution series is prepared using a reagent that develops color upon oxidation, such as TMBZ, and a solution of the nanoparticles of cerium oxide of the present invention of the same concentration is added to each dilution series to perform a color reaction with 3,3',5,5'-tetramethylbenzidine (TMBZ). The Michaelis-Menten equation is applied to this reaction to calculate the oxidase activity. The reaction rate is calculated from the change in absorbance over time at each TMBZ concentration, and the reciprocal of the reaction rate is plotted against the reciprocal of the concentration of TMBZ, which serves as a substrate. A straight line is obtained by plotting each substrate concentration, and the reciprocal of the y-intercept is used to determine the maximum reaction velocity Vₘₐₓ, which is then multiplied by the slope of the line to calculate the Michaelis-Menten constant Kₘ. The maximum reaction velocities Vₘₐₓ as a value indicating the oxidase activity are compared. Since TMBZ only dissolves in acidic conditions, the oxidase activity can be measured at different pH using a compound such as 2,2'-azinobis(3-ethylbenzothiazoline-6-sulfonic acid) (AzBTS) or N,N'-bis(2-hydroxy-3-sulfopropyl)tolidine (SAT-3) dopamine instead of TMBZ.

The nanoparticle of cerium oxide of the present invention, its dispersion, the resin composition or its molded product to which the nanoparticle or its dispersion is applied, or fibrous material to which the nanoparticle or its dispersion is applied can be used as the oxidizing agent. For example, by utilizing its oxidizing effect, it can be used as a homogeneous catalyst in organic synthesis reactions or polymerization, or as a wet etching solution for semiconductors. Alternatively, by utilizing its oxidizing effect, it can be used as a solution in place of an oxidase solution. Specifically, it can be used as a substitute for an oxidase or peroxidase solution in an antibody-antigen reaction or in a detection reaction utilizing nucleic acid hybridization or in tissue staining, or it can be used for electrochemical detection reaction by coating the nanoparticles of cerium oxide onto an electrode for immobilization. **In** addition, it can be used as a bleaching agent/disinfectant that utilizes its oxidizing action to break down and remove dirt, odors, allergens, bacteria, fungi, and mold. Specifically, it can be used as a bleaching agent to clean clothes, tableware, kitchens, toilets, washrooms, bathrooms, medical equipment, etc. Examples of the cleaning method include soaking, and spraying with a spray, a humidifier, or a nebulizer. It can be added as a disinfectant to swimming pools, bathtubs, and hot springs, or it can be used as body soap, hand soap, disinfectant, gargling mouthwash, mouthwash, hand gel, disinfectant spray, germicidal spray, deodorizing spray, wet tissue, disinfectant wipes, etc. The nanoparticles of cerium oxide of the present invention may be allowed to remain on the object after the above cleaning or disinfection to maintain the deodorizing, antiviral, antibacterial, or antifungal effects. The performance of the oxidizing agent can be evaluated using, for example, a fading reaction of organic dyes, which will be described later.

The nanoparticle of cerium oxide of the present invention or its dispersion, when used as the oxidizing agent, can be used in combination with alcohol, a surfactant, a disinfectant, or a natural organic substance. Examples of the alcohols include ethanol and isopropanol, examples of the surfactant include sodium alkylbenzenesulfonate, polyoxyethylene alkyl ether, and alkyl glucoside, examples of the disinfectant include chlorhexidine, acrinol, melbromin, and crystal Violet, and examples of the natural organic substance include polyphenols, catechins, tannic acid, chitin, chitosan, isothiocyanates, hinokitiol, limonene, polylysine, terpenoids, saponins, flavonoids, and carotene. Several of them may be combined in use.

The nanoparticles of cerium oxide of the present invention or its dispersion, when used as the oxidizing agent, can be used in combination with another known oxidizing agent or cleaning agent, disinfectant, antiviral agent, or antibacterial agent. Examples include cationic disinfectants such as hypochlorous acid, sodium hypochlorite, povidone-iodine, oxidol, ozone water, peracetic acid, ethanol, isopropanol, 2-phenoxy ethanol, 1-phenoxypropanol, 2 phenoxypropanol, phenol, cresol, halogenated phenol, and quaternary ammonium salts (benzalkonium chloride), anionic disinfectants such as sodium alkyl benzene sulfonate, cleaning agents such as the polyoxyethylene alkyl ether, diclosan, triclosan, bis -(2-pyridylthio-1-oxide) zinc, polyhexamethylene biguanidine hydrochloride, 8-oxyquinoline, polylysine, etc. Several of them may be used in combination. Examples of the halogenated phenol include hexachlorophene, triclosan, trichlorophenol, tribromophenol, pentachlorophenol, dibromol, and their salts.

The nanoparticles of cerium oxide of the present invention or the dispersion can be added during the molding of fibers, tubes, beads, rubber, films, plastics, etc. as an additive to impart oxidation performance, antiviral performance, or antibacterial performance, or applied to the surfaces of these as the dispersion, or can be used for processing such as deodorization, antiallergy, antibacterial, antifungal, antiviral processing. Examples of products that can be processed by the nanoparticles of cerium oxide of the present invention or the dispersion include drain cover closures for kitchen sinks, drain plugs, packings for fixing window glass, packings for fixing mirrors, waterproof packings for bathrooms, sinks, and kitchens, refrigerator door lining packings, bath mats, non-slip rubber for washbasins and chairs, hoses, shower heads, packings used in water purifiers, plastic products for water purifiers, packings used in washing machines, plastic products for washing machines, masks, protective clothing, medical caps, medical shoe covers, air conditioner filters, air purifier filters, vacuum cleaner filters, ventilation fan filters, vehicle filters, air conditioner filters, plastic parts such as fins for air conditioner, louvers for air conditioner outlets, and ventilation fans; plastic parts such as fins for car air conditioner, louvers for car air conditioner outlets, and ventilation fans; clothing, bedding, nets such as nets for screen windows, nets for chicken coops, mosquito nets, wallpaper, windows, blinds, interior materials for buildings such as hospitals, interior materials for trains and automobiles, vehicle seats, blinds, chairs, sofas, facilities handling viruses, building materials such as doors, ceiling boards, floor panels, and windows. As described above, products processed by the nanoparticle of cerium oxide of the present invention or its dispersion can be used as sanitary materials in a variety of fields.

The nanoparticle of cerium oxide of the present invention or its dispersion has high antiviral performance. Further, the nanoparticle of cerium oxide of the present invention, its dispersion, the resin composition or its molded product to which the nanoparticle or its dispersion is applied, or fibrous material to which the nanoparticle or its dispersion is applied can be used as the antiviral agent, antiviral resin, or antiviral fiber. An example of the method of evaluating the performance as the antiviral agent is a method in which the nanoparticle of cerium oxide of the present invention, its dispersion, the resin composition or its molded product to which the nanoparticle or its dispersion is applied, or fibrous material to which the nanoparticle or its dispersion is applied, is brought into contact with or mixed with virus, and then the amount of virus is determined. Examples of the methods of quantifying the virus include a method of measuring the amount of virus antigen using the ELISA method, a method of quantifying the virus nucleic acid using PCR, a method of measuring the infectivity titer using the plaque method, and a method of measuring the infectivity titer using the 50% infectious dose measurement method. In the present invention, for the antiviral performance, a method of measuring the infectivity titer by the plaque method or the 50% infectious dose measurement method is preferably used. In the 50% infectious dose measurement method, the units for the infectivity titer are TCID₅₀ (tissue culture infectious dose 50) when tested on cultured cells, EID₅₀ (egg infectious dose 50) when tested on hatched chicken eggs, and LD₅₀ (lethal dose 50) when tested on animals. In the 50% infectious dose measurement method, the Reed-Muench, Behrens-Kaeber, and Spearman-Karber methods are used to calculate the infectivity titer from the data obtained. In the present invention, the Reed-Muench method is used. As for the criterion for determining the antiviral performance, generally the antiviral performance is determined to be effective when the logarithmic decrement value of the infectivity titer is 2.0 or more, with respect to the infectivity titer before application of the nanoparticle of cerium oxide of the present invention, its dispersion, or the resin composition or fibrous material to which the nanoparticle of cerium oxide of the present invention or its dispersion is applied, or with respect to a control without containing the nanoparticle of the present invention.

A suitable aspect of the dispersion that contains the nanoparticle of cerium oxide of the present invention acid contains condensed phosphoric acid or/and a salt thereof and the nanoparticle of the cerium oxide, and has the logarithmic decrement value of the virus infectivity titer TCID₅₀ in the 50% infectious dose measurement in the virus inactivation test on the cell culture of 2.0 or more with respect to the infectivity titer before application of the nanoparticle of cerium oxide of the present invention or with respect to a control without containing the nanoparticle of the present invention. Since the logarithmic decrement value of the virus infectivity titer TCID₅₀ in the virus inactivation test is 2.0 or more, it can be used as an antiviral agent. The logarithmic decrement values of the virus infectivity titer are preferably 2.5 or more, and particularly preferably 3.0 or more.

Examples of the virus that can be inactivated by the nanoparticle of cerium oxide of the present invention or its dispersion include rhinovirus, poliovirus, foot- and-mouth disease virus, rotavirus, norovirus, enterovirus, hepatovirus, astrovirus, sapovirus, hepatitis E virus, influenza A, B, C virus, parainfluenza virus, mumps virus (epidemic parotitis), measles virus, human metapneumovirus, RS virus, Nipah virus, Hendra virus, yellow fever virus, dengue virus, Japanese encephalitis virus, West Nile virus, hepatitis B, C virus, eastern and western equine encephalitis virus, O'nyong-nyong Virus, rubella virus, Lassa virus, Junin virus, Machupo virus, Guanarito virus, Sabia virus, Crimean-Congo hemorrhagic fever virus, sandfly fever, hantavirus, Sin Nombre virus, rabies virus, Ebola virus, Marburg virus, bat lyssavirus, human T-cell leukemia virus, human immunodeficiency virus, human coronavirus, SARS coronavirus, SARS coronavirus 2, human parvovirus, polyomavirus, human papillomavirus, adenovirus, herpesvirus, varicella-zoster virus, EB virus, cytomegalovirus, smallpox virus, monkeypox, cowpox virus, molluscipoxvirus, parapoxvirus.

The nanoparticle of cerium oxide of the present invention or its dispersion has high antibacterial performance. The nanoparticle of cerium oxide of the present invention, its dispersion, the resin composition or its molded product to which the nanoparticle or its dispersion is applied, or fibrous material to which the nanoparticle or its dispersion is applied can be used as an antibacterial agent, an antibacterial resin,antibacterial fiber.

An example of a method of evaluating the antibacterial performance is EN1040:2005, which is the European Norm (EN) European Standard Test Method. In this test method, the bacteria solution is added to the test solution containing the active component of the antibacterial agent, and the number of bacteria is measured after a certain time. The bacteria solution contains 0.85% NaCl and 0.1% tryptone as medium components and is mixed so that the volume ratio of test solution : bacterial solution is 9:1. As for the criterion for judging the antibacterial performance, when the logarithmic decrement value of the number of bacteria is 2.0 or higher with respect to the number of bacteria before application of the nanoparticle of cerium oxide of the present invention, or with respect to a control without containing the nanoparticle of the present invention, the antibacterial performance is judged to be effective. Examples of the method of quantifying the number of bacteria include a method of measuring the amount of bacterial cells by turbidity (OD600) measurement, a method of measuring the amount of bacterial cells by a colony formation method, and a method of quantifying the nucleic acid of bacterial cells by PCR. In the present invention, a method of measuring the infectivity titer by the turbidity measurement or colony formation method is preferably used for antibacterial performance.

A suitable aspect of the dispersion containing the nanoparticle of cerium oxide of the present invention contains condensed phosphoric acid or/and its salt and the nanoparticle of cerium oxide, and the logarithmic decrement value of the amount of bacterial cells is 2.0 or more with respect to the infectivity titer before application of the nanoparticle of cerium oxide of the present invention, or with respect to a control without containing the nanoparticle of the present invention. Since the logarithmic decrement value of the number of bacteria in the antibacterial test is 2.0 or more, it can be used as an antibacterial agent. The logarithmic decrement values of the number of bacteria are preferably 2.5 or more, and particularly preferably 3.0 or more.

Examples of microorganisms for which the nanoparticle of cerium oxide of the present invention or its dispersion exhibits the antibacterial performance include the following. Examples of bacteria include gram-positive bacteria and gram-negative bacteria. Examples of gram-negative bacteria include bacteria of the genus Escherichia such as Escherichia coli, bacteria of the genus Salmonella such as Salmonella enterica, bacteria of the genus Pseudomonas such as Pseudomonas aeruginosa, bacteria of the genus Shigella such as Shigella dysenteriae, bacteria of the genus Klebsiella such as Klebsiella pneumoniae, and bacteria of the genus Legionella such as Legionella pneumophila. Examples of gram-positive bacteria include bacteria of the genus Staphylococcus, such as Staphylococcus aureus, bacteria of the genus Bacillus, such as Bacillus subtilis, and bacteria of the genus Mycobacterium, such as Mycobacterium tuberculosis. Examples of fungi include fungi and yeasts. Examples of fungi include filamentous fungi of the genus Aspergillus such as Aspergillus niger, filamentous fungi of the genus Penicillium such as Penicillium notatum, filamentous fungi of the genus Cladosporium such as Cladosporium cladosporioides, filamentous fungi of the genus Alternaria such as Alternaria alternata, filamentous fungi of the genus Trichoderma such as Trichoderma viride, and filamentous fungi of the genus Chaetomium such as Chaetomium globosum. Examples of yeasts include yeasts of the genus Saccharomyces, such as baker's yeast and brewer's yeast, and yeasts of the genus Candida, such as Candida albicans.

The nanoparticle of cerium oxide of the present invention or its dispersion can be added to the disinfectant to impart an antiviral activity or antibacterial activity to the disinfectant solution. As the disinfectant, those containing as active ingredients chlorine-based, iodine-based, peroxide-based, aldehyde-based, phenol-based, biguanide-based, mercury-based, alcohol-based, anionic surfactant-based, cationic surfactant-based, amphoteric surfactant-based, non-ionic surfactant-based, naturally occurring substance-based, or other disinfecting components can be used. The nanoparticle of cerium oxide of the present invention or its dispersion to the liquid containing ultrafine bubbles can be added to impart an antiviral activity or antibacterial activity.

For liquid disinfectant, the concentration of the nanoparticle of cerium oxide of the present invention may be set arbitrarily between 0.0001 mass% and 10 mass%.

Examples of chlorine-based disinfecting component include sodium hypochlorite, chlorine, and chlorinated isocyanuric acid.

Examples of iodine-based disinfecting component include iodine, povidone-iodine, nonoxynol iodine, and phenoxy iodine.

Examples of peroxide-based disinfecting component include hydrogen peroxide, potassium permanganate, peracetic acid, organic peracid, sodium percarbonate, sodium perborate, and ozone.

Examples of aldehyde-based disinfecting component include glutaraldehyde, phthalal, and formaldehyde.

Examples of phenolic disinfecting component include isopropyl methylphenol, thymol, eugenol, triclosan, cresol, phenol, chlorocresol, para-chloromethacresol, para-chlorometaxylenol, orthophenylphenol, para-oxybenzoic acid alkyl esters, resorcinol, hexachlorophene, and salicylic acid or its salt.

Examples of biguanide-based disinfecting component include chlorhexidine, chlorhexidine gluconate, and chlorhexidine hydrochloride.

Examples of mercury-based disinfecting component include mercurochrome, mercuric chloride, and thimerosal.

Examples of the alcoholic disinfecting component include ethanol and isopropanol. The concentration of the alcoholic disinfecting component in this case may be 30 to 80 mass%.

Examples of the anionic surfactant-based disinfecting component include alkyl benzene sulfonates, fatty acid salts, higher alcohol sulfate ester salts, polyoxyethylene alkyl ether sulfate salts, α-sulfo fatty acid esters, α-olefin sulfonate salts, monoalkyl phosphate ester salts, alkane sulfonate salts, and others.

Examples of the cationic surfactant-based disinfecting component include alkyl trimethylammonium salts, dialkyl dimethylammonium salts, alkyl dimethyl benzylammonium salts, polyhexamethylene biguanide, and benzethonium chloride.

Examples of amphoteric surfactant-based disinfecting component include alkylamino fatty acid salts, alkyl betaines, and alkylamine oxides.

Examples of non-ionic surfactant-based disinfecting component include polyoxyethylene alkyl ether, polyoxyethylene/polyoxypropylene alkyl ether, polyoxyethylene/polyoxybutylene alkyl ether, alkyl amine ethoxylate, alkylamine alkoxylate, polyoxyethylene - polyoxypropylene block copolymer , polyoxyethylene - polyoxypropylene block copolymer (reverse type), ethylene oxide/propylene oxide adduct of polyhydric alcohol, alkyl glucoside, and fatty acid alkanol amide.

Examples of the naturally occurring disinfecting component include plant drugs such as hinokitiol, anethole, anise oil, borneol, camphor, carvone, cassia oil, chenopodium oil, cineole, citral, citronellal, eugenol, pinene, geraniol, lemon oil, liolol, menthol, orange oil, safrole, thymol, polyphenols (flavanols, gallotannins, ellagitannins, and phlorotannins), animal-based agents such as chitin and chitosan made from crustacean shells, calcined shell powder obtained by calcining scallop and oyster shells, and microbial drugs such as polylysine, microorganism-based agents such as polylysine, and enzyme-based agents such as lysozyme. In addition, antimicrobial peptides produced by organisms to defend themselves against external microorganisms can also be used, such as histatin, defensin, lactoferrin, lactoferricin that is a degradation product of lactoferrin, magainin, cecropin, melititin.

In addition, plant extracts can also be used as naturally occurring disinfecting components. Specific examples include plant extracts such as grapefruit seed extract; *Bassia scoparia* of Chenopodiaceae; blackberry lily of Iridaceae; Hypericum perforatum of Clusiaceae; Boswellia carterii, abies balsamea of Burseraceae; Adenophora triphylla of Campanulaceae; Echinacea purpure, Matricaria recutita, Arctium lappa, Solidago altissima, Atractylodes lancea of Asteraceae; Coptis japonica of Ranunculaceae; Lonicera japonica of Caprifoliaceae; Laurus nobilis of Lauraceae; Humulus lupulus of Moraceae; Scutellaria baicalensis, Origanum vulgare, schizonepeta spik, Salvia officinalis, thyme, garden balm, Mosla japonica, Lavandula angustifolia, Rosmarinus officinalis of Lamiaceae; amomum seed, Zingiber officinale of Zingiberaceae; Sambucus nigra of Caprifoliaceae; Cryptomeria japonica of Taxodiaceae; Angelica dahurica and Ledebouriella seseloides of Apiaceae; Polygonum aviculare of polygonaceae; Arctostaphylos uva-ursi of Ericaceae; Houttuynia cordata of Saururaceae; Tribulus terrestris of Zygophyllaceae; Causonis japonica of Vitaceae; allspice, Melaleuca alternifolia, eucalyptus, and Eugenia caryophyllata of Myrtaceae; Maackia amurensis, Sophora japonica, Sophora flavescens, Dalbergia cochinchinensis, and Millettia pendula of Fabaceae; Formosan sweetgum of Hamamelidaceae; Phellodendron amurense and Satsuma mandarin of Rutaceae; Russian comfrey of Boraginaceae; Barberries and nandina of Berberidaceae; Magnolia hypoleuca of Magnoliaceae; Sanguisorba officinalis and rose of Rosaceae; Viscum album of Viscaceae; Anemarrhena asphodeloides, *Aspidistra,* and *Hemerocallis fulva* of Liliaceae; Gentiana macrophylla of Gentianaceae; mousou bamboo of Gramineae; and Ascophyllum nodosum of Fucus.

Examples of ultrafine bubbles include bubbles having the particle diameter of 500 nm or less, containing one or two or more gases therein, selected from air, oxygen, hydrogen, nitrogen, carbon dioxide, argon, neon, xenon, fluorinated gases, ozone, and inert gases. Ultrafine bubbles are also called nanobubbles. The concentration may be 100,000 cells/ml or more.

The disinfectant containing the nanoparticles of cerium oxide of the present invention or its dispersion may include, in addition to the disinfecting component listed above, an appropriate optional component blended therein depending on the dosage form. Specifically, solvents, wetting agents, thickeners, antioxidants, pH adjusters, amino acids, preservatives, sweeteners, fragrances, surfactants, colorants, auxiliary agents to enhance the bactericidal effect, chelating agents, UV absorbers, defoaming agents, enzymes, formulation stabilizer, and the like may be contained.

The disinfectant including the nanoparticles of cerium oxide of the present invention or its dispersion added thereto can be provided in various forms such as liquid, gel, and powder. The liquid disinfectant can be provided, for example, as a lotion, spray, foam, and can be filled into, for example, a bottle with a measuring cap, a trigger-type spray container, or a squeeze-type or dispenser-type pump spray container, and sprayed or atomized for use. The liquid disinfectant can be impregnated into sheets of paper or cloth, filled into a container such as a bottle or bucket, and provided as wet wipes.

The nanoparticles of cerium oxide of the present invention or its dispersion can be added to a paint to impart the paint with oxidation performance, antiviral performance, and antibacterial performance. In this case, a resin emulsion composition may be included in the paint in order to immobilize the nanoparticles of cerium oxide of the present invention into the paint film.

The nanoparticles of the present invention has oxidation performance, antiviral performance, and antibacterial performance. On the other hand, since the nanoparticles of the present invention uses an anionic stabilizer, the nanoparticles of the present invention are characterized in that it does not impair dispersion stability of the resin emulsion before addition of the nanoparticles over a wide range of pH.

Examples of the resin emulsion composition include ethylene vinyl acetate resin emulsions, vinyl chloride resin emulsions, epoxy resin emulsions, acrylic resin emulsions, urethane resin emulsions, acrylic silicone resin emulsions, fluororesin emulsions, and synthetic resin emulsions composed of resin components such as composite thereof. The mass ratio of the nanoparticles of cerium oxide of the present invention to be added to the paint and solid content in the resin emulsion can be set arbitrarily between 0.01:99.99 and 99.99:0.01.

The ethylene-vinyl acetate copolymer resin emulsion is a copolymerized product of ethylene and vinyl acetate monomer, with which a vinyl monomer having a functional group such as an amino group, a secondary amino group, a tertiary amino group, a quaternary amino group, a carboxyl group, an epoxy group, a sulfonic acid group, a hydroxyl group, a methylol group, or an alkoxy acid group may be further copolymerized.

The vinyl chloride copolymer resin emulsion is a polymerized product of vinyl chloride, with which a vinyl monomer having a functional group such as an amino group, a secondary amino group, a tertiary amino group, a quaternary amino group, a carboxyl group, an epoxy group, a sulfonic acid group, a hydroxyl group, a methylol group, or an alkoxy acid group may be further copolymerized.

In the present specification, "(meth)" means that the reference to acrylic acid derivatives (including acrylamide and acrylonitrile) includes not only the acrylic acid derivatives, but also the corresponding methacrylic acid derivatives and any copolymers thereof.

Examples of the resins that can be used for the preparation of the epoxy resin emulsion include bisphenol resins. Examples of the bisphenol resins include, but are not particularly limited to, bisphenol A-type epoxy resin, bisphenol F-type epoxy resin, bisphenol AD-type epoxy resin, bisphenol S-type epoxy resin, flame retardant-type epoxy resins such as glycidyl ether of tetrabromobisphenol A, glycidyl ether-type epoxy resins of bisphenol A propylene oxide adduct , and hydrogenated bisphenol A (or F)-type epoxy resins. Examples of other epoxy resins include glycidyl ester-type epoxy resins, glycidylamine-type epoxy resins, novolac-type epoxy resins, fluorinated epoxy resins, rubber modified epoxy resins containing polybutadiene or NBR, p-oxybenzoic acid glycidyl ether ester-type epoxy resins, m-aminophenol-type epoxy resins, diaminodiphenylmethane-based epoxy resins, urethane modified epoxy resins with urethane bonds, various alicyclic epoxy resins, glycidyl ethers of polyhydric alcohol, hydantoin-type epoxy resins, epoxides of unsaturated polymers such as petroleum resins, and amino-containing glycidyl ether resins.

Examples of the monomer that can be used to prepare the acrylic resin emulsion include (meth)acrylate ester-based monomers such as methyl (meth)acrylate, ethyl (meth)acrylate, propyl (meth)acrylate, butyl (meth)acrylate, hexyl (meth)acrylate, heptyl (meth)acrylate, octyl (meth)acrylate, octadecyl (meth)acrylate, 2-ethylhexyl (meth)acrylate, cyclohexyl (meth)acrylate, nonyl (meth)acrylate, dodecyl (meth)acrylate, stearyl (meth)acrylate, isobornyl (meth)acrylate, dicyclopentanyl (meth)acrylate, phenyl (meth)acrylate, and benzyl (meth)acrylate;
unsaturated bond-containing monomers having a carboxyl group such as acrylic acid, methacrylic acid, β-carboxyethyl (meth)acrylate, 2-(meth)acryloyloxypropionic acid, crotonic acid, itaconic acid, maleic acid, fumaric acid, itaconic acid half ester, maleic acid half ester, maleic anhydride, and itaconic anhydride; glycidyl group-containing polymerizable monomers such as glycidyl (meth)acrylate and allyl glycidyl ether; hydroxyl group-containing polymerizable monomers such as 2-hydroxyethyl (meth)acrylate, 2-hydroxypropyl (meth)acrylate, polyethylene glycol mono(meth)acrylate, and glycerol mono(meth)acrylate; ethylene glycol di(meth)acrylate, 1,6-hexanediol di(meth)acrylate, neopentyl glycol di(meth)acrylate, trimethylolpropane tri(meth)acrylate, polyethylene glycol di(meth)acrylate, polypropylene glycol di(meth)acrylate, diallyl phthalate, divinylbenzene, and allyl (meth)acrylate.

Examples of the monomer that can be used to prepare the urethane resin emulsion include, as a polyisocyanate component, 2,4-tolylene diisocyanate, 2,6-tolylenediisocyanate, m-phenylene diisocyanate, p-phenylene diisocyanate, 4,4'-diphenyl methane diisocyanate, 2,4'-diphenyl methane diisocyanate, 2,2'-diphenyl methane diisocyanate, 3,3'-dimethyl-4,4'-biphenylene diisocyanate, 3,3'-dimethoxy-4,4'-biphenylene diisocyanate, 3,3'-dichloro-4,4'-biphenylene diisocyanate, 1,5-naphthalene diisocyanate, 1,5-tetrahydronaphthalene diisocyanate, tetramethylene diisocyanate, 1,6-hexamethylene diisocyanate, dodecamethylene diisocyanate, trimethylhexamethylene diisocyanate, 1,3-cyclohexylene diisocyanate, 1,4-cyclohexylene diisocyanate, xylylene diisocyanate, tetramethylxylylene diisocyanate, hydrogenated xylylene diisocyanate, lysine diisocyanate, isophorone diisocyanate, 4,4'-dicyclohexyl methane diisocyanate, and 3,3'-dimethyl-4,4'-dicyclohexyl methane diisocyanate. Examples of the diol component include polyester polyol, polyether polyol, polycarbonate polyol, polyacetal polyol, polyacrylate polyol, polyesteramide polyol, polythioether polyol, and polyolefin polyol such as polybutadiene-based polyolefin polyol.

Examples of the silicon-containing acrylic monomer that can be used to prepare the acrylic silicon resin emulsion include γ-(meth)acryloxypropyl trimethoxysilane, γ-(meth)acryloxypropyl triethoxysilane, γ-(meth)acryloxypropylmethyl dimethoxysilane, and γ-(meth)acryloxypropylmethyl diethoxysilane.

Examples of the monomer that can be used to prepare the fluororesin emulsion include fluorooresin (vinylidene fluoride, trifluoroethylene, chlorotrifluoroethylene, tetrafluoroethylene, pentafluoroethylene, hexafluoropropylene, etc.), and fluorine-containing (meth)acrylate (trifluoroethyl(meth)acrylate, pentafluoropropyl (meth)acrylate, perfluorocyclohexyl (meth)acrylate, etc.).

The paint containing the nanoparticle of cerium oxide of the present invention or its dispersion may contain pigments, matting materials, aggregates, fibers, cross-linkers, plasticizers, preservatives, mold inhibitors, antibacterial agents, defoamers, viscosity regulators, leveling agents, pigment dispersing agents, anti-settling agents, anti-sagging agents, UV absorbers, light stabilizers, antioxidants, adsorbents, etc. as needed. These components can be blended in paint compositions alone or in combination.

The paint to which the nanoparticle of cerium oxide of the present invention or its dispersion is added can be used, for example, to paint the interior surface of buildings. Examples of the interior surfaces include mortar, concrete, gypsum board, siding board, extruded board, slate, asbestos cement board, fiber-mixed cement board, calcium silicate board, ALC board, metal, wood, glass, rubber, ceramic, fired tile, porcelain tile, plastic, synthetic resin, and other base materials, cloth, wallpaper, or coating films formed on these base materials. It can also be applied to exterior surfaces of buildings and structures other than buildings.

The nanoparticle of the present invention can be prepared as a resin composition containing the nanoparticle by adding the cerium oxide particle of the present invention or the dispersion containing the cerium oxide particle to the resin which serves as a base (the base resin). The resin composition exhibits oxidative decomposition performance against the deleterious material such as virus and bacteria. When the nanoparticle produced through a step of the hydrothermal treatment, the resin composition, which is characterized by having more excellent color tone can be obtained.

The type of the base resin is not limited and may be a thermoplastic resin, a thermosetting resin, a homopolymer, a copolymer, or a blend of two or more types of polymers. From the viewpoint of good moldability, thermoplastic resins are preferred.

Examples of thermoplastic resins that can be used include polyolefins such as polyethylene, polypropylene, polystyrene, and polymethylpentene, alicyclic polyolefins, styrene-based resins such as acrylonitrile styrene resin (AS resin), acrylonitrile butadiene styrene resin (ABS resin), polyamides such as nylon 6 and the nylon 66, polyesters such as polyethylene terephthalate, polytrimethylene terephthalate, polybutylene terephthalate, and polybutylene succinate, polycarbonate, polyarylate, polyacetal, polyphenylene sulfide, vinyl chloride, fluorine-based resins such as tetrafluoroethylene, trifluoroethylene, trifluoroethylene chloride, tetrafluoroethylene-hexafluoropropylene copolymer, and vinylidene fluoride, aramid, polyimide, acrylic, methacrylic, polyacetal, polyglycolic acid, and polylactic acid. Examples of the thermosetting resin that can be used include phenol resin, epoxy resin, urea resin, melamine resin, unsaturated polyester, polyurethane, polyimide, and silicone resin.

The resin composition of the present invention is obtained by adding the cerium oxide nanoparticle of the present invention to the base resin, but the method of addition is not particularly limited. For example, it may be added to a base resin that has been molten by heating or the like and then kneaded, or it may be mixed with a base resin in a specified ratio and then melt-kneaded, or surface treatmed cerium oxide particle may be added to the base resin together with an additive such as a flame retardant, a plasticizer, an antistatic agent, an antioxidant, a light fastness agent, a hydrolysis inhibitor, a pigment, or a lubricant. **In** addition, by exposing and unevenly distributing the cerium oxide nanoparticle of the present invention on the resin surface, the contact efficiency with the deleterious material such as virus or bacteria, is improved, thereby exerting a sufficient effect. The method of uneven distribution is not particularly limited, but in a case of using the additive in combination, the efficiency of uneven distribution can be increased by selecting the additive, which has a relatively higher affinity with the cerium oxide nanoparticle of the present invention relative to the base resin. Examples of the additive includes higher fatty acids, acid esters, acid amides, higher alcohols, and low and high molecular weight polymers of the surfactant, which may be preferably applied without limitation as long as they have affinity with the cerium oxide nanoparticle of the present invention. They may be added alone or in combination of two or more. The nanoparticle may also be added by adhering the nanoparticle of the present invention or its dispersion after the base resin has been molded. **In** this case, the additive may be used to efficiently adhere the nanoparticle to the molded base resin. Examples of the additive include binder resins such as acrylic resins, epoxy resins, melamine resins, urethane resins, polyamide resins, polyimide resins, polyester resins, urea resins, phenol resins, and silicone resins. When the dispersion is added by adhering it to the base resin, the liquid component contained in the dispersion is preferably removed after addition of the nanoparticle. **In** the method of removing liquid components contained in the dispersion, air drying, drying by heating, or vacuum drying may be performed as a drying step.

The cerium oxide nanoparticle of the present invention to be added to the base resin can be melt-kneaded by known methods in any form, including powder, pellets, slurry, dispersion in water, dispersion in an organic solvent, without any particular limitation. The cerium oxide nanoparticle of the present invention may be added to the base resin together with a dispersing agent or as a mixture with a dispersing agent. The dispersing agent of the cerium oxide nanoparticle of the present invention is not particularly limited, but a cationic surfactant such as quaternary ammonium salts, anionic the surfactant such as higher fatty acid salts and alkyl sulfate ester salts, amphoteric surfactant such as alkyl betaines, and
non-ionic surfactant such as polyoxyethylene sorbitan fatty acid salts and polyoxyethylene alkyl ether are all applicable. the cationic surfactant or the non-ionic surfactant,
but preferably the cationic surfactant or the non-ionic surfactant are all applicable, and cationic surfactants or non-ionic surfactants are more preferable. The mixing ratio of the dispersing agent to the cerium oxide nanoparticle of the present invention is not particularly limited and can be arbitrarily adjusted as long as the compatibility with the base resin to be added and the oxidation performance are not significantly impaired.

The content percentage of cerium oxide nanoparticle of the present invention relative to the entire resin composition of the present invention is not limited as long as the deleterious materials such as viruses and bacteria, can be decomposed, but is preferably 0.01 mass% or more and 60 mass% or less. When the content percentage is less than 0.01 mass%, the effect will not be sufficient, and when it is more than 60 mass%, the mechanical properties, such as strength and durability, of the resin may be impaired. Preferably, it is 0.05 mass% or more and 50 mass% or less. More preferably, it is 0.1 mass% or more and 30 mass% or less. Still more preferably, it is 3 mass% or more and 10 mass% or less. The resin composition of the present invention may be kneaded with the resin which is the same as or different from the base resin in a given ratio as a master batch. When used as a master batch, the content percentage of the cerium oxide nanoparticle of the present invention is preferably 10 mass% or more.

Examples of the method of producing the resin composition of the present invention include, but are not particularly limited to, a method of mixing components constituting the resin composition using a mixer, and a method of uniformly melt-kneading components constituting the resin composition. Examples of the mixer include a V-shaped blender, a super mixer, a super floater, and a Henschel mixer. The melt-kneading temperatures is preferably 200°C to 320°C, and ore preferably 200°C to 300°C. The obtained resin composition can be pelletized using a pelletizer for use.

The resin composition of the present invention may be molded by an arbitrary molding method. Examples of the molding method include injection molding, extrusion molding, inflation molding, blow molding, vacuum molding, compression molding, and gas-assisted molding.

The resin composition of the present invention is characterized by excellent color tone when using cerium oxide nanoparticle produced through the hydrothermal treatment step. The yellowness index value of the resin composition which contains 1 mass% of the nanoparticle produced through the hydrothermal treatment of the present invention is preferably 15 or less, more preferably 10 or less, and even more preferably 5 or less. The yellowness index used herein is a value measured using a color computer (manufactured by Suga Test Instruments Co., Ltd.) in accordance with JIS-K7373.

The resin composition of the present invention can be widely used as the molded product of any shape. Examples of the molded product include injection molded products, extrusion molded products, vacuum/pressure molded products, blow molded products, sheets, fibers, fabrics, non-woven fabrics, or composites with other materials.

The resin composition of present invention can be used as a raw material to produce, for example, resin products such as automobile interior materials, electrical appliance casings, hanging straps, handrails, doorknobs, partition boards, and paints.

The fibrous material containing the cerium oxide nanoparticle of the present invention can be obtained by immobilizing the cerium oxide nanoparticles of the present invention onto the fiber base material, or by spinning the resin composition with the cerium oxide nanoparticle of the present invention kneaded therein. The method of immobilizing the cerium oxide nanoparticles of the present invention on the fiber base material is preferred because the cerium oxide nanoparticles are exposed on the surface of the resulting the fibrous material, making it easier to exert the antiviral performance and the antibacterial performance.

An example of the method of immobilizing the cerium oxide nanoparticle of the present invention on the fiber base material is a method of immobilization by a dipping method, a spraying method, or a coating method, using cerium oxide particles of the present invention or a dispersion containing the cerium oxide particles.

In a case of immobilizing the cerium oxide nanoparticles of the present invention to the aforementioned fiber base material, the addition of a binder component to the dispersion that serves as an adhesive with the fiber base material is preferred because it prevents the cerium oxide nanoparticle from falling off from the fiber base material.

Types of the binder components including, but are not limited to, acrylic resins, epoxy resins, melamine resins, urethane resins, polyamide resins, polyimide resins, polyester resins, urea resins, phenol resins, and silicone resins may be preferably applied.

In a case of immobilizing the cerium oxide nanoparticles of the present invention on the aforementioned fiber base material, the additive may be added to the dispersion to control the dispersibility and viscosity of the cerium oxide nanoparticles of the present invention.

As an additive, a surfactant is preferred. Cationic surfactants such as quaternary ammonium salts, anionic the surfactant such as higher fatty acid salts and alkyl sulfates, amphoteric the surfactant such as alkyl betaines, and the non-ionic surfactant such as polyoxyethylene sorbitan fatty acid salts and the polyoxyethylene alkyl ether are all applicable, but more preferably the cationic surfactant or the non-ionic surfactant.

The mixing ratio of the additives to the cerium oxide nanoparticles of the present invention is not particularly limited as long as the antiviral performance and the antibacterial performance are not significantly impaired, and can be adjusted arbitrarily.

When spinning with the resin composition of the present invention, thermoplastic resin is preferred as the base resin. The method of spinning is, for example, to make the resin composition of the present invention into a molten polymer and guide it through a piping to a spinning pack. Polymer introduced from a polymer inlet of the spinning pack passes through a filter layer composed of a filtering medium and a filter, and is discharged from the discharge hole of the spinneret to obtain a fiber.

The type of the fiber base material is not limited and may be any of natural fibers, synthetic fibers, or inorganic fibers, or may be a mixed fiber or composite fiber of two or more of these types. Examples of natural fibers that can be preferably used include, but are not limited to, cellulosic fibers such as cotton, hemp, and rayon, and animal fibers such as wool, silk, and down. Examples of synthetic fibers that can be preferably used include, but are not limited to, polyolefin fibers, polyester fibers, polyamide fibers, acrylic fibers, polyurethane fibers, and polyvinyl alcohol fibers. Examples of inorganic fibers that can be preferably used include, but are not limited to, glass fibers, carbon fibers, and ceramic fibers. It can also be preferably applied to fibers that have been processed to have an irregular cross-section or to be hollow. Examples of the form of fibers that can be preferably used include, but are not limited to, thread, woven fabric, and non-woven fabric.

When the nanoparticle produced through the hydrothermal treatment step is used to obtain a fibrous material, the fibrous material and the fibrous product obtained from the fibrous material as a raw material, are characterized by low colorability.

The content percentage of cerium oxide nanoparticle of the present invention relative to the entire fibrous material of the present invention is not limited as long as the deleterious materials such as viruses and bacteria can be decomposed, but is preferably 0.01 mass% or more and 60 mass% or less. When the content percentage is less than 0.01 mass%, the effect will not be sufficient, and when it is more than 60 mass%, the mechanical properties, such as strength and durability, of the fiber may be impaired, or the breathability when made into a cloth may be impaired. The content percentage is preferably 0.05 mass% or more and 50 mass% or less, more preferably 0.1 mass% or more and 30 mass% or less, and still more preferably 3 mass% or more and 10 mass% or less.

The fibrous material of the present invention obtained in this way is characterized by its oxidative decomposition performance against deleterious materials such as viruses and bacteria.

The fibrous material of the present invention can be used as a raw material for fibrous products such as masks, protective clothing, filters, mats, chairs, gowns, lab coats, curtains, sheets, automobile interior material, wipes.

### Examples

The present invention will be explained in more detail with reference to Examples below.

### <Materials and Methods>

Cerium nitrate (III) hexahydrate, sodium tripolyphosphate, sodium hexametaphosphate, and 30 mass% hydrogen peroxide solution were obtained from FUJIFILM Wako Pure Chemical Corporation, sodium pyrophosphate was obtained from Alfa Aesar, sodium trimetaphosphate was obtained from Merck, and TMBZ·HCl was obtained from DOJINDO LABORATORIES. The commercially available cerium oxide dispersion (289744) used in Comparative Examples was obtained from Merck. Amicon Ultra 15 (10 kD) used for purification was purchased from Merck Millipore.

Other reagents were purchased from FUJIFILM Wako Pure Chemical Corporation, Tokyo Kasei Corporation, or Sigma-Aldrich Japan and used as is without any particular purification.

For the measurements of the hydrodynamic diameter of nanoparticle of cerium oxide, Zeta-potential & Particle size Analyzer ELSZ-2000ZS manufactured by Otsuka Electronics Co., Ltd. was used, and SpectraMax iD3 manufactured by MOLECULAR DEVICE was used as a plate reader for absorbance measurement.

### (Example 1) Preparation of dispersion of nanoparticle of cerium oxide using pyrophosphoric acid as stabilizer

Fifty milliliters of water was added to a beaker to dissolve 0.54 g of pyrophosphoric acid. Two milliliters of a 10 mass% aqueous solution of cerium (III) nitrate hexahydrate was added and the mixture was stirred at room temperature for 10 minutes. Thereafter, 2 ml of a 1.2 mass% aqueous solution of hydrogen peroxide was added dropwise and reacted at room temperature overnight. The reaction solution was purified by the ultrafiltration membrane with a molecular weight cutoff of 10 kD to obtain an orange-colored dispersion containing nanoparticles of cerium oxide.

### (Example 2) Preparation of dispersion of nanoparticle of cerium oxide using tripolyphosphoric acid as stabilizer

The reaction was performed under the same conditions as in Example 1, except that 0.54 g of sodium tripolyphosphate was used instead of 0.54 g of sodium pyrophosphate, to obtain an orange-colored dispersion containing nanoparticles of cerium oxide.

### (Example 3) Preparation of dispersion of nanoparticle of cerium oxide using trimetaphosphoric acid as stabilizer

The reaction was performed under the same conditions as in Example 1, except that 0.54 g of sodium trimetaphosphate was used instead of 0.54 g of sodium pyrophosphate, to obtain an orange-colored dispersion containing nanoparticles of cerium oxide.

### (Example 4) Preparation of dispersion of nanoparticle of cerium oxide using hexametaphosphoric acid as stabilizer

The reaction was performed under the same conditions as in Example 1, except that 0.54 g of sodium hexametaphosphate was used instead of 0.54 g of sodium pyrophosphate, to obtain an orange-colored dispersion containing nanoparticles of cerium oxide.

### (Example 5) Preparation of dispersion of nanoparticle of cerium oxide by hydrothermal treatment using pyrophosphoric acid as stabilizer

The dispersion obtained in Example 1 was transferred to a pressure-resistant container, and the hydrothermal treatment was performed at 120°C (199 kPa) for 20 minutes to obtain a dispersion of nanoparticles of cerium oxide with pyrophosphoric acid as a stabilizer. The resulting dispersion was pale brown.

### (Example 6) Preparation of dispersion of nanoparticle of cerium oxide by hydrothermal treatment using tripolyphosphoric acid as stabilizer

The dispersion obtained in Example 2 was transferred to a pressure-resistant container, and the hydrothermal treatment was performed at 120°C (199 kPa) for 20 minutes to obtain a dispersion of nanoparticles of cerium oxide with tripolyphosphoric acid as a stabilizer. The resulting dispersion was pale brown.

### (Example 7) Preparation of dispersion of nanoparticle of cerium oxide by hydrothermal treatment using trimetaphosphoric acid as stabilizer

The dispersion obtained in Example 3 was transferred to a pressure-resistant container, and the hydrothermal treatment was performed at 120°C (199 kPa) for 20 minutes to obtain a dispersion of nanoparticles of cerium oxide with trimetaphosphoric acid as a stabilizer. The resulting dispersion was pale brown.

### (Example 8) Preparation of dispersion of nanoparticle of cerium oxide by hydrothermal treatment using hexametaphosphoric acid as stabilizer

The dispersion obtained in Example 4 was transferred to a pressure-resistant container, and the hydrothermal treatment was performed at 120°C (199 kPa) for 20 minutes to obtain a dispersion of nanoparticles of cerium oxide with hexametaphosphoric acid as a stabilizer. The resulting dispersion was pale brown.

### (Comparative Example 1) Trial to prepare dispersion of nanoparticle of cerium oxide using phosphoric acid as stabilizer

The reaction was performed under the same conditions as in Example 1, except that 0.54 g of phosphoric acid or 0.54 g of disodium hydrogenphosphate was used instead of 0.54 g of sodium pyrophosphate. All of the reaction liquids yielded a white precipitate and failed to disperse nanoparticles of cerium oxide.

Patent Literature 2 (Japanese Translation of PCT International Application Publication No. 2005-500436) discloses a production method including post-addition of a stabilizer. The paragraphs [0021] to [0022] list various phosphoric acid compounds as the stabilizer, and the paragraph [0023] exemplifies phosphoric acid as a preferred stabilizer. In the production method of the present invention, however, phosphoric acid cannot be used as a stabilizer. From these results, it can be inferred the nanoparticle of cerium oxide, obtained by the method including post-addition of the stabilizer disclosed in Patent Literature 2, has different properties from the nanoparticle of the present invention.

### (Comparative Example 2) Preparation of dispersion of nanoparticle of cerium oxide by post-addition of phosphoric acid

With reference to Patent Literature 2 (Japanese Translation of PCT International Application Publication No. 2005-500436), a dispersion was prepared by a production method including post-addition of phosphoric acid to a dispersion of cerium oxide nanoparticle for adsorption. Commercially available 20 wt% dispersion of nanoparticle of cerium oxide (Merck, 289744) was diluted to 1 wt%, and 0.1 g of phosphoric acid was added to 20 ml of the diluted solution and stirred at room temperature for 2 hours. Thereafter, the reaction solution was purified by the ultrafiltration membrane with a molecular weight cutoff of 10 kD to obtain a dispersion containing nanoparticles of cerium oxide.

### (Comparative Example 3) Preparation of dispersion of nanoparticle of cerium oxide by post-addition of pyrophosphoric acid

The reaction was performed under the same conditions as in Comparative Example 2, except that 0.1 g of sodium pyrophosphate was used instead of 0.1 g of phosphoric acid, to obtain a dispersion containing nanoparticles of cerium oxide.

### (Comparative Example 4) Preparation of dispersion of nanoparticle of cerium oxide by post-addition of tripolyphosphoric acid

The reaction was performed under the same conditions as in Comparative Example 2, except that 0.1 g of sodium tripolyphosphate was used instead of 0.1 g of phosphoric acid, to obtain a dispersion containing nanoparticles of cerium oxide.

### (Comparative Example 5) Preparation of dispersion of nanoparticle of cerium oxide by post-addition of trimetaphosphoric acid

The reaction was performed under the same conditions as in Comparative Example 2, except that 0.1 g of sodium trimetaphosphate was used instead of 0.1 g of phosphoric acid, to obtain a dispersion containing nanoparticles of cerium oxide.

### (Comparative Example 6) Preparation of dispersion of nanoparticle of cerium oxide by post-addition of hexametaphosphoric acid

The reaction was performed under the same conditions as in Comparative Example 2, except that 0.1 g of sodium hexametaphosphate was used instead of 0.1 g of phosphoric acid, to obtain a dispersion containing nanoparticles of cerium oxide.

### (Example 9) Measurement of hydrodynamic diameter of nanoparticle of cerium oxide

The hydrodynamic diameter of the nanoparticles of cerium oxide prepared in Examples 1 to 8 were measured by dynamic light scattering (DLS). The solvent used in the measurement was water, and the average particle diameter (cumulant diameter) of the hydrodynamic diameter was obtained. The values obtained are shown in Table 1.

The average particle diameter ranged from 11.2 to 51.4 nm, which confirmed that all of them were nanoparticles.

### (Example 10) Measurement of zeta potential of dispersion containing nanoparticle of cerium oxide

The zeta potential of the nanoparticles of cerium oxide produced in Examples 1 to 8 were measured. The solvent for the measurement was water, and each sample was adjusted to pH 7 with nitric acid or sodium hydroxide. The values obtained are shown in Table 1.

The zeta potential ranged from -37.7 to -46.6 mV, which confirmed that all nanoparticles had high negative charge.

**[Table 1]**

| Dispersion | Stabilizer | Solution | Example 9 | Example |
|---|---|---|---|---|
| | | | Particle diameter | 10 Zeta potential |
| Example 1 | Pyrophosphoric acid | Orange | 23.2 nm | -37.7 mV |
| Example 2 | Tripolyphosphoric acid | Orange | 11.2 nm | -37.8 mV |
| Example 3 | Trimetaphosphoric acid | Orange | 11.3 nm | -39.3 mV |
| Example 4 | Hexametaphosphoric acid | Orange | 9.2 nm | -46.3 mV |
| Example 5 | Pyrophosphoric acid (Hydrothermal treatment) | Pale brown | 51. 4 nm | -38.9 mV |
| Example 6 | Tripolyphosphoric acid (Hydrothermal treatment) | Pale brown | 26.5 nm | -37.8 mV |
| Example 7 | Trimetaphosphoric acid (Hydrothermal treatment) | Pale brown | 26.9 nm | -40.3 mV |
| Example 8 | Hexametaphosphoric acid (Hydrothermal treatment) | Pale brown | 13.0 nm | -46.6 mV |

### (Example 11) Dispersion stability of nanoparticle of cerium oxide

The dispersion stability of the cerium oxide nanoparticles produced in Examples 1, 2, 5, and 6 were evaluated. Each sample was adjusted to pH 3 to 9 with nitric acid or sodium hydroxide. The dispersion stability was evaluated based on the occurrence of precipitation after centrifugation at 10,000 G for 2 minutes at a concentration of 0.1wt %.

The results are shown in Table 2. When no precipitate was visually observed, the dispersion was determined to be stable ('absent' in the table), whereas when a precipitate was visually observed, the dispersion was determined to be unstable ('present' in the table). All the nanoparticle showed high dispersion stability over a wide pH range.

### [Table 2]

**[Table 2]**

| Dispersion | Stabilizer | Example 11 pH/presence or absence of precipitate | | | | | | |
|---|---|---|---|---|---|---|---|---|
| | | 3 | 4 | 5 | 6 | 7 | 8 | 9 |
| Example 1 | Pyrophosphoric acid | Absent | Absent | Absent | Absent | Absent | Absent | Absent |
| Example 2 | Tripolyphosphoric acid | Absent | Absent | Absent | Absent | Absent | Absent | Absent |
| Example 5 | Pyrophosphoric acid (Hydrothermal treatment) | Absent | Absent | Absent | Absent | Absent | Absent | Absent |
| Example 6 | Tripolyphosphoric acid (Hydrothermal treatment) | Absent | Absent | Absent | Absent | Absent | Absent | Absent |

### (Example 12) Measurement of APHA of dispersion containing nanoparticle of cerium oxide

The nanoparticles of cerium oxide produced in Examples 5 to 8 which involve hydrothermal treatment were adjusted to prepare a 0.1 mass% dispersion, and APHA was measured. The results are shown in Table 3.

The APHA ranged from 41 to 94, which confirmed that all the nanoparticles had low colorability.

### (Reference Example 1) Measurement of APHA of dispersion containing nanoparticle of cerium oxide

The nanoparticles of cerium oxide produced in Comparative Examples 2 to 6 without hydrothermal treatment were adjusted to prepare a 0.1 mass% dispersion, and APHA was measured. The results are shown in Table 3.

### (Example 13) Measurement of APHA of dispersion containing nanoparticle of cerium oxide

The nanoparticles of cerium oxide produced in Examples 1 to 4 without hydrothermal treatment were adjusted to prepare a 0.1 mass% dispersion, and APHA was measured. The results are shown in Table 3

The APHA was 500 or more. It was observed that the hydrothermal treatment improved the colorability of the nanoparticle compared to Example 12.

**[Table 3]**

| | Dispersion | Stabilizer | 0.1wt%DispersionAPHA |
|---|---|---|---|
| Example 12 | Example 5 | Pyrophosphoric acid (Hydrothermal treatment) | 94 |
| | Example 6 | Tripolyphosphoric acid (Hydrothermal treatment) | 41 |
| | Example 7 | Trimetaphosphoric acid (Hydrothermal treatment) | 47 |
| | Example 8 | Hexametaphosphoric acid (Hydrothermal treatment) | 45 |
| Reference Example 1 | Comparative Example 2 | Phosphoric acid (Post-addition) | 500 or more |
| | Comparative Example 3 | Pyrophosphoric acid (Post-addition) | 500 or more |
| | Comparative Example 4 | Tripolyphosphoric acid (Post-addition) | 500 or more |
| | Comparative Example 5 | Trimetaphosphoric acid (Post-addition) | 500 or more |
| | Comparative Example 6 | Hexametaphosphoric acid (Post-addition) | 500 or more |
| Example 13 | Example 1 | Pyrophosphoric acid | 500 or more |
| | Example 2 | Tripolyphosphoric acid | 500 or more |
| | Example 3 | Trimetaphosphoric acid | 500 or more |
| | Example 4 | Hexametaphosphoric acid | 500 or more |

### (Example 14) Measurement of oxidation performance by TMBZ

The oxidation performance was calculated as an oxidase activity by a method using an oxidation color-developing reagent (TMBZ) described in A. Asati, Angew. Chem. Int. Ed. 2009, 48, 2308 - 2312.

TMBZ·HCl was dissolved in water to prepare a dilution series of 10, 5, 2.5, 1.25, and 0.625 mM. 160 µl of 50 mM citrate buffer (pH 4) and 20 µl of TMBZ solution of each concentration were mixed and added to a 96-well plate, to which was added 20 µl of an aqueous solution of the nanoparticle of cerium oxide of the present invention of Examples 1 to 8 prepared to 1 mg/ml, and immediately set in a plate reader to measure the change over time in absorbance at 652 nm associated with the blue coloration of TMBZ. The measurement interval was 30 seconds and the measurement period was 10 minutes.

The Michaelis-Menten equation was applied to calculate the oxidase activity. In the oxidase activity measurements, since there was no change in the nanoceria concentration, a steady-state approximation was performed to calculate the Michaelis-Menten constant Kₘ and the maximum reaction velocity Vₘₐₓ. The Michaelis-Menten equation was analyzed using a Lineweaver plot (double reciprocal plot). Among the results obtained, the maximum reaction velocity Vₘₐₓ, the values representing the oxidase activity, are shown in Table 4.

### (Comparative Example 7) Measurement of oxidation performance by TMBZ

The oxidase activity measurements were performed on the nanoparticle of cerium oxide prepared in Comparative Examples 2 to 6 under similar operations and conditions, and the maximum reaction velocity Vₘₐₓ was calculated and shown in Table 4.

A comparison of Example 14 and Comparative Example 7 confirmed that the nanoparticle of cerium oxide of the present invention has a higher oxidase activity. Due to such properties, the nanoparticle of cerium oxide of the present invention can be used as the oxidizing agent.

On the other hand, the oxidase activity was lower in the nanoparticles of cerium oxide of Comparative Examples 2 to 6 compared to the nanoparticles of cerium oxide of Examples 1 to 8.

**[Table 4]**

| | Dispersion | Stabilizer | Oxidase activity Vₘₐₓ [µM/sec] |
|---|---|---|---|
| Example 14 | Example 1 | Pyrophosphoric acid | 2.1 |
| | Example 2 | Tripolyphosphoric acid | 2.4 |
| | Example 3 | Trimetaphosphoric acid | 2.3 |
| | Example 4 | Hexametaphosphoric acid | 2.5 |
| | Example 5 | Pyrophosphoric acid (Hydrothermal treatment) | 2.5 |
| | Example 6 | Tripolyphosphoric acid (Hydrothermal treatment) | 2.5 |
| | Example 7 | Trimetaphosphoric acid (Hydrothermal treatment) | 2.7 |
| | Example 8 | Hexametaphosphoric acid (Hydrothermal treatment) | 2.9 |
| Comparative Example 7 | Comparative Example 2 | Phosphoric acid (Post-addition) | 0.1 |
| | Comparative Example 3 | Pyrophosphoric acid (Post-addition) | 0.2 |
| | Comparative Example 4 | Tripolyphosphoric acid (Post-addition) | 0.1 |
| | Comparative Example 5 | Trimetaphosphoric acid (Post-addition) | 0.1 |
| | Comparative Example 6 | Hexametaphosphoric acid (Post-addition) | 0.2 |

### (Example 15) Virus inactivation test

0.1 ml of the virus solution (Influenza virus, ATCC, VR-1679, Influenza A virus (H3N2)) was mixed with 0.9 ml of the dispersion of nanoparticles of cerium oxide prepared in Examples 1 to 8 which had been adjusted to 5 mg/ml, and allowed to act for 1 hour. Then, PBS was added as a stopping solution to stop the action on the virus. The solution was used as a stock solution for a sample for the viral titer measurement and the infectivity titer was measured using the plaque measurement method. The logarithmic decrement value of the infectivity titer with respect to the infectivity titer before application of the nanoparticle of cerium oxide is shown in Table 5.

### (Comparative Example 8) Virus inactivation test

The same operations as in Example 15 were repeated except that the nanoparticles of cerium oxide of Comparative Examples 2 to 6 were used, and the logarithmic decrement value of the infectivity titer is shown in Table 5.

A comparison of Example 15 and Comparative Example 8 confirmed that the nanoparticle of the present invention had a high antiviral activity. Due to such properties, the nanoparticle of cerium oxide of the present invention can be used as the antiviral agent.

On the other hand, the antiviral activity was lower in the nanoparticles of cerium oxide of Comparative Examples 2 to 6 compared to the nanoparticles of cerium oxide of Examples 1 to 8.

**[Table 5]**

| | Dispersion | Stabilizer | Logarithm decrement of infectivity titer |
|---|---|---|---|
| Example 15 | Example 1 | Pyrophosphoric acid | 4.1 or more |
| | Example 2 | Tripolyphosphoric acid | 4.1 or more |
| | Example 3 | Trimetaphosphoric acid | 4.1 or more |
| | Example 4 | Hexametaphosphoric acid | 4.1 or more |
| | Example 5 | Pyrophosphoric acid (Hydrothermal treatment) | 4.1 or more |
| | Example 6 | Tripolyphosphoric acid (Hydrothermal treatment) | 4.1 or more |
| | Example 7 | Trimetaphosphoric acid (Hydrothermal treatment) | 4.1 or more |
| | Example 8 | Hexametaphosphoric acid (Hydrothermal treatment) | 4.1 or more |
| Comparative Example 8 | Comparative Example 2 | Phosphoric acid (Post-addition) | 0.5 |
| | Comparative Example 3 | Pyrophosphoric acid (Post-addition) | 1.2 |
| | Comparative Example 4 | Tripolyphosphoric acid (Post-addition) | 1.4 |
| | Comparative Example 5 | Trimetaphosphoric acid (Post-addition) | 1.3 |
| | Comparative Example 6 | Hexametaphosphoric acid (Post-addition) | 1.5 |

### (Example 16) Antimicrobial test against Escherichia coli

Escherichia coli precultured in LB medium was suspended in a bacterial suspension preparation (0.1% tryptone, 0.85% NaCl) to prepare a 10⁸ CFU/ml of bacterial suspension. 0.1 ml of this bacterial suspension was mixed with 0.9 ml of a 1 mg/ml dispersion of nanoparticles of cerium oxide prepared in Examples 1 to 8, the cerium oxide, and the mixture was left to stand at room temperature for one hour. Thereafter, the mixture was used as a stock solution to prepare a dilution series, which was then inoculated onto LB agar medium to measure the colony count. The logarithmic decrement values of the colony count relative to the colony count before acting the nanoparticle of cerium oxide are shown in Table 6 as antimicrobial activity values.

### (Comparative Example 9) Antimicrobial test against Escherichia coli

The same operations as in Example 16 were repeated except that the nanoparticles of cerium oxide of Comparative Examples 2 to 6 were used, and the antibacterial activity value is shown in Table 6.

A comparison of Example 16 and Comparative Example 9 confirmed that the nanoparticle of the present invention has a higher antibacterial activity. Due to such properties, the nanoparticle of cerium oxide of the present invention can be used as the antibacterial agent.

On the other hand, the antibacterial activity was lower in the nanoparticles of cerium oxide of Comparative Examples 2 to 6 compared to the nanoparticles of cerium oxide of Examples 1 to 8.

**[Table 6]**

| | Dispersion | Stabilizer | Antibacterial activity |
|---|---|---|---|
| Example 16 | Example 1 | Pyrophosphoric acid | 4.0 |
| | Example 2 | Tripolyphosphoric acid | 4.1 |
| | Example 3 | Trimetaphosphoric acid | 4.5 |
| | Example 4 | Hexametaphosphoric acid | 4.3 |
| | Example 5 | Pyrophosphoric acid (Hydrothermal treatment) | 3.2 |
| | Example 6 | Tripolyphosphoric acid (Hydrothermal treatment) | 3.1 |
| | Example 7 | Trimetaphosphoric acid (Hydrothermal treatment) | 3.6 |
| | Example 8 | Hexametaphosphoric acid (Hydrothermal treatment) | 3.6 |
| Comparative Example 9 | Comparative Example 2 | Phosphoric acid (Post-addition) | 0.3 |
| | Comparative Example 3 | Pyrophosphoric acid (Post-addition) | 0.6 |
| | Comparative Example 4 | Tripolyphosphoric acid (Post-addition) | 0.7 |
| | Comparative Example 5 | Trimetaphosphoric acid (Post-addition) | 0.7 |
| | Comparative Example 6 | Hexametaphosphoric acid (Post-addition) | 0.8 |

### (Example 17) Preparation of resin composition (ABS resin)

The dispersion of the nanoparticles of cerium oxide produced in Example 2 was lyophilized at -45°C, 20 Pa. 99 parts by mass of ABS resin pellets (manufactured by Toray Industries, Inc., general-purpose resin "TOYOLAC (registered trademark)" 100-322), 1 part by mass of nanoparticles of cerium oxide obtained by lyophilization, and 0.5 parts by mass of pure water as a spreading agent were blended using a Henschel mixer at 230°C for 60 seconds. Then, the resulting mixture was melt-kneaded at an extrusion temperature of 230°C using a 40 mmφ vented extruder, and extruded into a gut shape to pelletize, thereby to obtain a resin composition. The resulting pellets were then molded into a square plate having a thickness of 3 mm using an injection molding machine with a cylinder temperature set at 230°C.

### (Example 18) Preparation of resin composition (ABS resin)

The resin composition was obtained and a square plate was formed under the same conditions as in Example 17, except that the dispersion of the nanoparticle of cerium oxide produced in Example 4 was used instead of the dispersion of the nanoparticle of cerium oxide produced in Example 2.

### (Example 19) Preparation of resin composition (ABS resin)

The resin composition was obtained and a square plate was formed under the same conditions as in Example 17, except that the dispersion of the nanoparticle of cerium oxide produced in Example 6 was used instead of the dispersion of the nanoparticle of cerium oxide produced in Example 2.

### (Example 20) Preparation of resin composition (ABS resin)

The resin composition was obtained and a square plate was formed under the same conditions as in Example 17, except that the dispersion of the nanoparticle of cerium oxide produced in Example 8 was used instead of the dispersion of the nanoparticle of Example 2.

### (Example 21) Preparation of resin composition (Nylon 6 resin)

The dispersion of the nanoparticles of cerium oxide produced in Example 2 was lyophilized at -45°C, 20 Pa. 99 parts by mass of Nylon 6 resin pellets (manufactured by Toray Industries, Inc.) and 1 part by mass of nanoparticle of cerium oxide obtained by lyophilization was blended, melt-kneaded using a 40 mmφ vented extruder at an extrusion temperature of 250°C, and extruded into a gut shape to obtain a pelletized resin composition. The resulting pellets were then molded into a square plate having a thickness of 3 mm using an injection molding machine with a cylinder temperature set at 250°C.

### (Example 22) Preparation of resin composition (Nylon 6 resin)

The resin composition was obtained and a square plate was formed under the same conditions as in Example 21, except that the dispersion of the nanoparticle of cerium oxide produced in Example 6 was used instead of the dispersion of the nanoparticle of cerium oxide produced in Example 2.

### (Example 23) Preparation of resin composition (PBT resin)

99 parts by mass of polybutylene terephthalate (PBT) resin pellets (manufactured by Toray Industries, Inc.) and 1 part by mass of a dispersion of nanoparticle of cerium oxide obtained in Example 2 was blended, melt-extruded using a 40 mmφ vented extruder at an extrusion temperature of 250°C, and extruded into a gut shape to obtain a pelletized resin composition. The resulting pellets were then molded into a square plate having a thickness of 3 mm using an injection molding machine with a cylinder temperature set at 250°C.

### (Example 24) Preparation of resin composition (PBT resin)

The resin composition was obtained and a square plate was formed under the same conditions as in Example 23, except that the dispersion of the nanoparticle of cerium oxide produced in Example 6 was used instead of the dispersion of the nanoparticle of cerium oxide produced in Example 2.

### (Comparative Example 10) Preparation of resin composition (ABS resin)

The resin composition was obtained and a square plate was formed under the same conditions as in Example 17, except that the dispersion of the nanoparticle of cerium oxide produced in Comparative Example 4 was used instead of the dispersion of the nanoparticle of cerium oxide produced in Example 2.

### (Comparative Example 11) Preparation of resin composition (ABS resin)

The resin composition was obtained and a square plate was formed under the same conditions as in Example 17, except that the dispersion of the nanoparticle of cerium oxide produced in Comparative Example 6 was used instead of the dispersion of the nanoparticle of cerium oxide produced in Example 2.

### (Comparative Example 12) Preparation of resin composition (Nylon 6 resin)

The resin composition was obtained and a square plate was formed under the same conditions as in Example 21, except that the dispersion of the nanoparticle of cerium oxide produced in Comparative Example 4 was used instead of the dispersion of the nanoparticle of cerium oxide produced in Example 2.

### (Comparative Example 13) Preparation of resin composition (PBT resin)

The resin composition was obtained and a square plate was formed under the same conditions as in Example 23, except that the dispersion of the nanoparticle of cerium oxide produced in Comparative Example 4 was used instead of the dispersion of the nanoparticle of cerium oxide produced in Example 2.

### (Example 25) Virus inactivation test

The virus inactivation test was performed as follows. The molded products of the resin compositions of Examples 17 to 24 as 50 mm × 50 mm × 1 mm square plates were placed in moist petri dishes. 0.4 ml of the virus solution (Influenza virus, ATCC, VR-1679, Influenza A virus (H3N2)) was dropped onto the molded product of the resin composition, and allowed to act for 24 hours with a film of 4 cm × 4 cm film (made of PP) placed thereon. Then, SDLP was added as a stopping solution to stop the action on the virus, and the virus on the molded product of the resin composition was washed out and recovered. The recovered solution was used as a stock solution for a sample for the viral titer measurement and the infectivity titer was measured using the TCID₅₀ method.

The difference between the common logarithm of the infectivity titer of the virus when tested using the molded product of the resin composition of the present invention, and the common logarithm of the infectivity titer of the virus when tested using the resin composition (blank) which does not use the nanoparticle of cerium oxide, was used as the virus inactivation index to evaluate antiviral activity. The higher virus inactivation index indicates the higher antiviral activity. Specifically, the antiviral performance was determined to be effective when the logarithmic decrement value of the infectivity titer (the virus inactivity index) was 2.0 or more.

Evaluation results are shown in Table 7.

### (Comparative Example 14) Virus inactivation test

The molded product of the resin composition produced in Comparative Examples 10 to 13, was subjected to the virus inactivation test with the same operations and conditions as in Example 25, and the antiviral activity is shown in Table 7.

The resin compositions of Examples 17 to 24 showed a higher antiviral activity compared to the resin compositions of Comparative Examples 10 to 13.

**[Table 7]**

| | | Base resin | Dispersion | Antiviral activity |
|---|---|---|---|---|
| Example 25 | Example 17 | ABS | Example 2 | 2.2 |
| | Example 18 | ABS | Example 4 | 2.6 |
| | Example 19 | ABS | Example 6 | 2.3 |
| | Example 20 | ABS | Example 8 | 2.8 |
| | Example 21 | Nylon6 | Example 2 | 2.5 |
| | Example 22 | Nylon6 | Example 6 | 2.4 |
| | Example 23 | PBT | Example 2 | 2.3 |
| | Example 24 | PBT | Example 6 | 2.2 |
| Comparative Example 14 | Comparative Example 10 | ABS | Comparative Example 4 | 0.4 |
| | Comparative Example 11 | ABS | Comparative Example 6 | 0.3 |
| | Comparative Example 12 | Nylon6 | Comparative Example 4 | 0.6 |
| | Comparative Example 13 | PBT | Comparative Example 4 | 0.4 |

### (Example 26) Measurement of YI

The color tone of the square plates obtained by Examples 19, 20, 22, and 24, including the nanoparticles of cerium oxide, which have been subjected to the hydrothermal treatment, was measured as yellowness index (YI) using a color computer manufactured by Suga Test Instruments Co., Ltd. to measure YI value. Evaluation results are shown in Table 8.

### (Example 27) Measurement of YI

The color tone of the square plates obtained by Examples 17, 18, 21, and 23, including the nanoparticles of cerium oxide, which have not been subjected to the hydrothermal treatment, was measured as yellowness index (YI) using a color computer manufactured by Suga Test Instruments Co., Ltd. to measure YI value. Evaluation results are shown in Table 8.

Comparing Example 26 and Example 27, it was observed that the resin composition produced using the nanoparticles of cerium oxide that have been subjected to the hydrothermal treatment had a reduced YI, i.e., an improved color tone.

**[Table 8]**

| | | Base resin | Dispersion | YI |
|---|---|---|---|---|
| Example 26 | Example 19 | ABS | Example 6 | 3.8 |
| | Example 20 | ABS | Example 8 | 3.5 |
| | Example 22 | Nylon6 | Example 6 | 4.9 |
| | Example 24 | PBT | Example 6 | 2.9 |
| Example 27 | Example 17 | ABS | Example 2 | 19.1 |
| | Example 18 | ABS | Example 4 | 19.2 |
| | Example 21 | Nylon6 | Example 2 | 34.4 |
| | Example 23 | PBT | Example 2 | 22.3 |

### (Example 28) Preparation of non-woven fabric (spunbond-type non-woven fabric made of polypropylene)

The dispersion of the nanoparticles of cerium oxide produced in Example 2 was lyophilized at -45°C, 20 Pa. The mass of the nanoparticle thus obtained was measured, and the concentration of the nanoparticle contained in the dispersion was determined.

The dispersion of the nanoparticle of cerium oxide produced in Example 2 was concentrated or diluted with water based on the concentration of the nanoparticle in the above dispersion to adjust the dispersion in water to contain 3 parts by mass of the nanoparticle, 3 parts by mass of self-crosslinking acrylic binder (VONCOAT AN-1170, manufactured by DIC Corporation) and 94 parts by mass of water.

Spunbond-type non-woven fabric made of polypropylene (manufactured by Toray Industries, Inc.) was cut into 5 cm squares and immersed in the above dispersion in water for 1 hour. Then, after lightly squeezing, the fabric was dried in an oven at 130°C for 2 hours. The resulting non-woven fabric with the cerium oxide nanoparticles immobilized thereon was visually observed for coloration.

### (Example 29) Preparation of non-woven fabric (spunbond-type non-woven fabric made of polypropylene)

A non-woven fabric with cerium oxide nanoparticles immobilized thereon was prepared in the same manner as in Example 28, except that the dispersion of the nanoparticle of cerium oxide produced in Example 4 was used instead of the dispersion of the nanoparticle of cerium oxide produced in Example 2.

### (Example 30) Preparation of non-woven fabric (spunbond-type non-woven fabric made of polypropylene)

A non-woven fabric with cerium oxide nanoparticles immobilized thereon was prepared in the same manner as in Example 28, except that the dispersion of the nanoparticle of cerium oxide produced in Example 6 was used instead of the dispersion of the nanoparticle of cerium oxide produced in Example 2.

### (Example 31) Preparation of non-woven fabric (spunbond-type non-woven fabric made of polypropylene)

A non-woven fabric with cerium oxide nanoparticles immobilized thereon was prepared in the same manner as in Example 28, except that the dispersion of the nanoparticle of cerium oxide produced in Example 8 was used instead of the dispersion of the nanoparticle of cerium oxide produced in Example 2.

### (Example 32) Preparation of non-woven fabric (rayon non-woven fabric)

A non-woven fabric with cerium oxide nanoparticles immobilized thereon was obtained in the same manner as in Example 28, except that a rayon non-woven fabric (manufactured by Kuraray Kuraflex Co., Ltd.) was used instead of the spunbond-type non-woven fabric made of polypropylene (manufactured by Toray Industries, Inc.).

### (Example 33) Preparation of non-woven fabric (rayon non-woven fabric)

A non-woven fabric with cerium oxide nanoparticles immobilized thereon was prepared in the same manner as in Example 32, except that the dispersion of nanoparticles of cerium oxide produced in Example 6 was used instead of the dispersion of nanoparticles of cerium oxide produced in Example 2.

### (Comparative Example 15) Preparation of non-woven fabric (spunbond-type non-woven fabric made of polypropylene)

A non-woven fabric with cerium oxide nanoparticles immobilized thereon was prepared in the same manner as in Example 28, except that the dispersion of nanoparticles of cerium oxide produced in Comparative Example 4 was used instead of the dispersion of nanoparticles of cerium oxide produced in Example 2.

### (Comparative Example 16) Preparation of non-woven fabric (spunbond-type non-woven fabric made of polypropylene)

A non-woven fabric with cerium oxide nanoparticles immobilized thereon was prepared in the same manner as in Example 28, except that the dispersion of nanoparticles of cerium oxide produced in Comparative Example 6 was used instead of the dispersion of nanoparticles of cerium oxide produced in Example 2.

### (Comparative Example 17) Preparation of non-woven fabric (rayon non-woven fabric)

A non-woven fabric with cerium oxide nanoparticles immobilized thereon was prepared in the same manner as in Example 32, except that the dispersion of nanoparticles of cerium oxide produced in Comparative Example 4 was used instead of the dispersion of nanoparticles of cerium oxide produced in Example 2.

### (Example 34) Virus inactivation test

The virus inactivation test was performed as follows. The non-woven fabrics of Examples 28 to 33 were cut into 20 mm x 20 mm and 0.4 g pieces were placed in vials. 0.2 ml of the virus solution (Influenza virus, ATCC, VR-1679, Influenza A virus (H3N2)) was dropped onto the non-woven fabric, and allowed to act for 2 hours. Then, SDLP was added as a stopping solution to stop the action on the virus, and the virus was washed out and recovered from the non-woven fabric. The recovered solution was used as a stock solution for a sample for the viral titer measurement and the infectivity titer was measured using the TCID₅₀ method.

The difference between the common logarithm of the infectivity titer of the virus when tested using the fibrous material of the resin composition of the present invention, and the common logarithm of the infectivity titer of the virus when tested using the resin composition (blank) which does not use the nanoparticle of cerium oxide, was used as the virus inactivation index to evaluate antiviral activity. The higher virus inactivation index indicates the higher antiviral activity. Specifically, the antiviral performance was determined to be effective when the logarithmic decrement value of the infectivity titer (the virus inactivity index) was 2.0 or more.

Evaluation results are shown in Table 9.

### (Comparative Example 18) Virus inactivation test

The fibrous materials produced in Comparative Examples 15 to 17 were subjected to the virus inactivation test with the same operations and conditions as in Example 34, and the antiviral activity is shown in Table 9.

The fibrous materials of Examples 28 to 33 showed a higher antiviral activity compared to the resin compositions of Comparative Examples 15 to 17.

**[Table 9]**

| | | Base fiber | Dispersion | Antiviral activity |
|---|---|---|---|---|
| Example 34 | Example 28 | Polypropylene | Example 2 | 2.9 |
| | Example 29 | Polypropylene | Example 4 | 2.7 |
| | Example 30 | Polypropylene | Example 6 | 2.5 |
| | Example 31 | Polypropylene | Example 8 | 2.9 |
| | Example 32 | Rayon | Example 2 | 3.5 |
| | Example 33 | Rayon | Example 6 | 3.6 |
| Comparative Example 18 | Comparative Example 15 | Polypropylene | Comparative Example 4 | 1.2 |
| | Comparative Example 16 | Polypropylene | Comparative Example 6 | 0.8 |
| | Comparative Example 17 | Rayon | Comparative Example 4 | 0.9 |

### (Example 35) Confirmation of colorability

The colorability of the non-woven fabrics obtained in Examples 30, 31, and 33, containing the nanoparticles of cerium oxide that had not been subjected to the hydrothermal treatment was visually observed. The results are shown in Table 10.

### (Example 36) Confirmation of colorability

The colorability of the non-woven fabrics obtained in Examples 28, 29, and 32, containing the nanoparticles of cerium oxide that had not been subjected to the hydrothermal treatment was visually observed. The results are shown in Table 10.

Comparing Example 35 and Example 36, it was observed that the fibrous material produced using the nanoparticles of cerium oxide that have been subjected to the hydrothermal treatment had a reduced YI, i.e., an improved color tone.

**[Table 10]**

| | | Base resin | Dispersion | Polypropylene |
|---|---|---|---|---|
| Example 35 | Example 30 | Polypropylene | Example 6 | Absent |
| | Example 31 | Polypropylene | Example 8 | Absent |
| | Example 33 | Rayon | Example 6 | Absent |
| Example 36 | Example 28 | Polypropylene | Example 2 | Present |
| | Example 29 | Polypropylene | Example 4 | Present |
| | Example 32 | Rayon | Example 2 | Present |

### (Example 37) Preparation of dispersion of nanoparticle of cerium oxide using tetrapolyphosphoric acid as stabilizer

The reaction was performed under the same conditions as in Example 1, except that 0.54 g of sodium tetrapolyphosphate was used instead of 0.54 g of sodium pyrophosphate, to obtain an orange-colored dispersion containing nanoparticles of cerium oxide.

### (Example 38) Preparation of dispersion of nanoparticle of cerium oxide using pentapolyphosphoric acid as stabilizer

The reaction was performed under the same conditions as in Example 1, except that 0.54 g of sodium pentapolyphosphate was used instead of 0.54 g of sodium pyrophosphate, to obtain an orange-colored dispersion containing nanoparticles of cerium oxide.

### (Example 39) Preparation of dispersion of nanoparticle of cerium oxide using polyphosphoric acid as stabilizer

The reaction was performed under the same conditions as in Example 1, except that 0.54 g of sodium polyphosphate (Wako Pure Chemical Corporation, 194-05935) was used instead of 0.54 g of sodium pyrophosphate, to obtain an orange-colored dispersion containing nanoparticles of cerium oxide.

### (Example 40) Preparation of dispersion of nanoparticle of cerium oxide using metaphosphoric acid as stabilizer

The reaction was performed under the same conditions as in Example 1, except that 0.54 g of sodium metaphosphate (Wako Pure Chemical Corporation, 199-08165) was used instead of 0.54 g of sodium pyrophosphate, to obtain an orange-colored dispersion containing nanoparticles of cerium oxide.

### (Example 41) Preparation of dispersion of nanoparticle of cerium oxide by hydrothermal treatment using tetrapolyphosphoric acid as stabilizer

The dispersion obtained in Example 1 was transferred to a pressure-resistant container, and the hydrothermal treatment was performed at 120°C (199 kPa) for 20 minutes to obtain a dispersion of nanoparticles of cerium oxide with tetrapolyphosphoric acid as a stabilizer. The resulting dispersion was pale brown.

### (Example 42) Preparation of dispersion of nanoparticle of cerium oxide by hydrothermal treatment using pentapolyphosphoric acid as stabilizer

The dispersion obtained in Example 1 was transferred to a pressure-resistant container, and the hydrothermal treatment was performed at 120°C (199 kPa) for 20 minutes to obtain a dispersion of nanoparticles of cerium oxide with pentapolyphosphoric acid as a stabilizer. The resulting dispersion was pale brown.

### (Example 43) Preparation of dispersion of nanoparticle of cerium oxide by hydrothermal treatment using polyphosphoric acid as stabilizer

The dispersion obtained in Example 1 was transferred to a pressure-resistant container, and the hydrothermal treatment was performed at 120°C (199 kPa) for 20 minutes to obtain a dispersion of nanoparticles of cerium oxide with polyphosphoric acid as a stabilizer. The resulting dispersion was pale brown.

### (Example 44) Preparation of dispersion of nanoparticle of cerium oxide by hydrothermal treatment using metaphosphoric acid as stabilizer

The dispersion obtained in Example 1 was transferred to a pressure-resistant container, and the hydrothermal treatment was performed at 120°C (199 kPa) for 20 minutes to obtain a dispersion of nanoparticles of cerium oxide with metaphosphoric acid as a stabilizer. The resulting dispersion was pale brown

### (Comparative Example 19) Dispersion containing nanoparticle of cerium oxide obtained by post-addition of hexametaphosphoric acid to the nanoparticle of cerium oxide with polyacrylic acid as stabilizer

With reference to Non-Patent Literature 1, the nanoparticles of anionic cerium oxide with polyacrylic acid as a stabilizer were produced using a production method using the oxidizing agent similar to the present invention. To 10 ml of a 1 mass% aqueous solution of sodium polyacrylate, 200 µl of a 10 mass% aqueous solution of cerium (III) nitrate hexahydrate was added and stirred for 5 minutes at room temperature. Thereafter, 200 µl of a 1.2 mass% aqueous solution of hydrogen peroxide was added, and heated to 40°C to react for 1 hour. The reaction solution was purified with the ultrafiltration membrane of 30 kD to obtain an orange-colored dispersion containing nanoparticles of cerium oxide. As with Comparative Example 6, 0.1 g of the hexametaphosphoric acid was added to 20 ml of the dispersion prepared to 1 wt%, and the mixture was stirred at room temperature for 2 hours. Thereafter, the reaction solution was purified by the ultrafiltration membrane with a molecular weight cutoff of 10 kD to obtain a dispersion containing nanoparticles of cerium oxide.

### (Example 45) Measurement of ratio of elemental phosphorus to elemental cerium (P/Ce ratio) by XPS

The ratios of elemental phosphorus to elemental cerium (P/Ce ratios) of the nanoparticles of cerium oxide obtained in Examples 1, 2, 4, 5, 6, and 8 were measured by the X-ray photoelectron spectroscopy (XPS). In the measurement, the excited X-ray was monocheomatic AlK_{α1,2} rays (1486.6eV), the X-ray diameter was 200 µm, and the photoelectron escape angle was 45°. The resulting spectrum was subjected to horizontal correction so that the P2p main the peak was 133.0 eV. In the measurements, the dispersions of the nanoparticles of cerium oxide manufactured in Examples 1, 2, 4, 5, 6, and 8 were placed in glass containers and dried with hot air at 120°C. The resulting powder of the nanoparticles of cerium oxide were ground in a mortar. The values obtained are shown in Table 11.

The results show that the nanoparticles of cerium oxide produced by Examples 1, 2, 4, 5, 6, and 8 have a high phosphorus ratio, with the ratio of elemental phosphorus to elemental cerium (P/Ce ratio) being 0.72 or more.

**[Table 11]**

| | Dispersion | Stabilizer | P/Ce |
|---|---|---|---|
| Example 45 | Example 1 | Pyrophosphoric acid | 1.88 |
| | Example 2 | Tripolyphosphoric acid | 6.35 |
| | Example 4 | Hexametaphosphoric acid | 5.00 |
| | Example 5 | Pyrophosphoric acid (Hydrothermal treatment) | 0.80 |
| | Example 6 | Tripolyphosphoric acid (Hydrothermal treatment) | 1.12 |
| | Example 8 | Hexametaphosphoric acid (Hydrothermal treatment) | 0.82 |

### (Comparative Example 20) Measurement of ratio of elemental phosphorus to elemental cerium (P/Ce ratio) by XPS

The ratios of elemental phosphorus to elemental cerium (P/Ce ratios) of the nanoparticles of cerium oxide obtained in Comparative Examples, 3, 4, 6, and 19 were measured by the X-ray photoelectron spectroscopy method (XPS). Measurements were performed under the same conditions as the Example 45. The values obtained are shown in Table 12.

The results show that the nanoparticles of cerium oxide produced by Comparative Examples, 3, 4, 6, and 19 have a low phosphorus ratio, with the ratio of elemental phosphorus to elemental cerium (P/Ce ratio) being 0.71 or less.

**[Table 12]**

| | Dispersion | Stabilizer | P/Ce |
|---|---|---|---|
| Comparative Example 20 | Comparative Example 3 | Pyrophosphoric acid (Post-addition) | 0.34 |
| | Comparative Example 4 | Tripolyphosphoric acid (Post-addition) | 0.33 |
| | Comparative Example 6 | Hexametaphosphoric acid (Post-addition) | 0.32 |
| | Comparative Example 19 | Hexametaphosphoric acid (Post-addition) | 0.71 |

### (Example 46) Measurement of molar ratio of Ce⁴⁺ to total Ce⁴⁺ and Ce³⁺ by XPS

Molar ratios of Ce⁴⁺ and Ce³⁺ of nanoparticles of cerium oxide obtained in Examples 1, 2, 4, 5, 6, and 8 were measured by X-ray photoelectron spectroscopy(XPS). In the measurement, the excited X-ray was monocheomatic AlKα1,2 rays (1486.6eV), the X-ray diameter was 200 µm, and the photoelectron escape angle was 45°. The resulting spectrum was subjected to horizontal correction so that the P2p main the peak was 133.0 eV. In the measurements, the dispersions of the nanoparticles of cerium oxide produced in Examples 1, 2, 4, 5, 6, and 8 were placed in glass containers and dried with hot air at 120°C. The resulting powder of the nanoparticle of cerium oxide was ground in a mortar. The values obtained are shown in Table 13.

The results show that the nanoparticle of cerium oxide produced by Examples 1, 2, 4, 5, 6, and 8 has a molar ratio of Ce⁴⁺ to the total Ce⁴⁺ and Ce³⁺ of 0.070 or more and 0.89 or less.

**[Table 13]**

| | Dispersion | Stabilizer | Ce⁴⁺ | Ce³⁺ |
|---|---|---|---|---|
| Example 46 | Example 1 | Pyrophosphoric acid | 20 | 80 |
| | Example 2 | Tripolyphosphoric acid | 45 | 55 |
| | Example 4 | Hexametaphosphoric acid | 81 | 19 |
| | Example 5 | Pyrophosphoric acid (Hydrothermal treatment) | 60 | 40 |
| | Example 6 | Tripolyphosphoric acid (Hydrothermal treatment) | 65 | 35 |
| | Example 8 | Hexametaphosphoric acid (Hydrothermal treatment) | 86 | 14 |

### (Comparative Example 21) Measurement of molar ratio of Ce⁴⁺ to total Ce⁴⁺ and Ce³⁺ by XPS

The molar ratios of Ce⁴⁺ and Ce³⁺ of the nanoparticles of cerium oxide obtained in Comparative Examples 3, 4, 6, and 19 were measured by the X-ray photoelectron spectroscopy (XPS). Measurements were performed under the same conditions as the Example 46. The values obtained are shown in Table 14.

The results show that a molar ratio of Ce⁴⁺ and Ce³⁺ of the nanoparticles of cerium oxide of Comparative Examples 3, 4, 6, and 19 is not 86:14 to 20:80.

**[Table 14]**

| | Dispersion | Stabilizer | Ce⁴⁺ | Ce³⁺ |
|---|---|---|---|---|
| Comparative Example 21 | Comparative Example 3 | Pyrophosphoric acid (Post-addition) | 90 | 10 |
| | Comparative Example 4 | Tripolyphosphoric acid (Post-addition) | 90 | 10 |
| | Comparative Example 6 | Hexametaphosphoric acid (Post-addition) | 90 | 10 |
| | Comparative Example 19 | Hexametaphosphoric acid (Post-addition) | 6 | 94 |

### (Example 47) XRD analysis of nanoparticle of cerium oxide

The dispersions of the nanoparticles of cerium oxide produced in Examples 5, 6, and 8 were placed in glass containers and dried with hot air at 120°C. The resulting powder of the nanoparticle of cerium oxide was ground in a mortar, and measured by X-ray diffraction (XRD). The measurement conditions were as follows: light source was CuKα ray, output was 40 kV, 40mA, detector was LynxEye, and the measurement range was 2θ = 5 to 90°. The obtained diffraction peak data for the XRD spectrum are shown in Table 15.

**[Table 15]**

| | Example 5 | Example 6 | Example 8 |
|---|---|---|---|
| Stabilizer | Pyrophosphoric acid (Hydrothermal treatment) | Tripolyphosphoric acid (Hydrothermal treatment) | Hexametaphosphoric acid (Hydrothermal treatment) |
| Example 47 XRD peak position (2θ/deg.) | 14.360 | 14.474 | 28.563 |
| | 19.897 | 19.902 | 31.341 |
| | 25.803 | 26.141 | 33.087 |
| | 28.560 | 29.080 | 42.021 |
| | 31.340 | 31.460 | 47.242 |
| | 33.079 | 37.679 | 56.267 |
| | 37.679 | 42.020 | 69.509 |
| | 42.079 | 48.559 | 76.829 |
| | 47.560 | 51.702 | 78.499 |
| | 52.239 | 56.160 | 88.143 |
| | 56.360 | 59.320 | |
| | 59.341 | 68.341 | |
| | 62.858 | 71.078 | |
| | 71.658 | | |

The results revealed that the nanoparticle of Example 5 had the diffraction peaks at 2θ = 31.340° and 42.079°, the nanoparticle of Example 6 had the diffraction peaks at 2θ = 31.460° and 42.020°, and the nanoparticle in Example 8 had the diffraction peaks at 2θ = 31.341° and 42.021°. The results show that the nanoparticle of cerium oxide of the present invention has the diffraction peaks in the regions of the Bragg angle 2θ of 30.5° or more and 32.0° or less, and 41.5° or more and 43.0° or less in the XRD spectrum.

### (Comparative Example 22) XRD analysis of nanoparticle of cerium oxide

The XRD measurements were performed with the same operation as in Example 47 on the nanoparticles of cerium oxide of Comparative Examples 3, 4, and 6 as a comparison of Examples 5, 6, and 8. The obtained diffraction peak data for the XRD spectrum are shown in Table 16. The results showed that the nanoparticle of cerium oxide of Comparative Examples 3, 4, and 6 had no diffraction peaks in the ranges of the Bragg angle 2θ = 30.5° or more and 32.0° or less and 41.5° or more and 43.0° or less in the XRD spectrum.

**[Table 16]**

| | Comparative Example 3 | Comparative Example 4 | Comparative Example 6 |
|---|---|---|---|
| Stabilizer | Pyrophosphoric acid (Post-addition) | Tripolyphosphoric acid (Post-addition) | Hexametaphosphoric acid (Post-addition) |
| Comparative Example 22 XRD peak | 28.588 | 28.500 | 28.538 |
| | 33.122 | 33.020 | 33.066 |
| | 47.525 | 47.440 | 47.469 |
| position (2θ/deg.) | 56.383 | 56.241 | 56.329 |
| | 59.129 | 59.100 | 59.089 |
| | 69.454 | 69.359 | 69.394 |
| | 76.738 | 76.680 | 76.722 |
| | 79.114 | 79.101 | 79.084 |
| | 88.466 | 88.420 | 88.430 |

## Claims

1. A nanoparticle of cerium oxide comprising an inorganic acid adsorbed onto its surface, wherein a zeta potential at pH 7 is 0 mV or less.

2. A nanoparticle of cerium oxide comprising a phosphorus compound adsorbed thereto, wherein a molar ratio (P/Ce ratio) of elemental phosphorus to elemental cerium in the XPS measurement is 0.72 or more.

3. The nanoparticle of cerium oxide according to claim 2, wherein the phosphorus compound is condensed phosphoric acid.

4. The nanoparticle of cerium oxide according to claim 2 or 3, wherein the XRD spectrum has peaks in the ranges of 2θ = 30.5° or more and 32.0° or less and 41.5° or more and 43.0° or less.

5. A nanoparticle of cerium oxide comprising a phosphorus compound adsorbed thereto, wherein a molar ratio of Ce⁴⁺ to the total Ce⁴⁺ and Ce³⁺ in the XPS measurement is 0.070 or more and 0.89 or less.

6. The nanoparticle of cerium oxide according to claim 5, wherein the phosphorus compound is condensed phosphoric acid.

7. The nanoparticle of cerium oxide according to claim 5 or 6, wherein the XRD spectrum has peaks in the ranges of 2θ = 30.5° or more and 32.0° or less and 41.5° or more and 43.0° or less.

8. A nanoparticle of cerium oxide, produced by the following step:
Step a): a step in which an oxidizing agent is added to a solution containing condensed phosphoric acid or/and a salt thereof and cerium (III) ion.

9. The nanoparticle of cerium oxide according to claim 8, wherein the pH of the solution at the time of adding the oxidizing agent is 5 or more.

10. The nanoparticle of cerium oxide according to claim 8, wherein the condensed phosphoric acid is polyphosphoric acid or metaphosphoric acid.

11. The nanoparticle of cerium oxide according to claim 10, wherein the polyphosphoric acid is pyrophosphoric acid, tripolyphosphoric acid, tetrapolyphosphoric acid, pentapolyphosphoric acid, or hexapolyphosphoric acid.

12. The nanoparticle of cerium oxide according to claim 10, wherein the metaphosphoric acid is trimetaphosphoric acid, tetrametaphosphoric acid, pentametaphosphoric acid, or hexametaphosphoric acid.

13. The nanoparticle of cerium oxide according to claim 8, wherein the nanoparticle of cerium oxide is produced by steps further comprising:
Step b): a step in which the solution obtained in the step a) is subjected to hydrothermal treatment.

14. The nanoparticle of cerium oxide according to claim 8, wherein APHA of a dispersion containing 0.1 mass% of the nanoparticle is 400 or less.

15. The nanoparticle of cerium oxide according to claim 8, wherein a zeta potential at pH 7 is - 20 mV or less.

16. A dispersion comprising the nanoparticle of cerium oxide according to any one of claims 1 to 3, 5, 6, and 8 to 15.

17. An oxidizing agent comprising the nanoparticle of cerium oxide according to any one of claims 1 to 3, 5, 6, and 8 to 15.

18. An antiviral agent comprising the nanoparticle of cerium oxide according to any one of claims 1 to 3, 5, 6, and 8 to 15.

19. An antibacterial agent comprising the nanoparticle of cerium oxide according to any one of claims 1 to 3, 5, 6, and 8 to 15.

20. A resin composition comprising the nanoparticle of cerium oxide according to any one of claims 1 to 3, 5, 6, and 8 to 15.

21. A resin product comprising the resin composition according to claim 20.

22. The resin product according to claim 21, wherein the resin product is selected from the group consisting of an automobile interior material, an electrical appliance casing, a hanging strap, a handrail, a doorknob, a partition board, and a paint.

23. A fibrous material comprising the nanoparticle of cerium oxide according to any one of claims 1 to 3, 5, 6, and 8 to 15.

24. A fibrous product comprising the fibrous material according to claim 23.

25. The fibrous product according to claim 24, wherein the fibrous product is selected from the group consisting of a mask, protective clothing, a filter, a mat, a chair, a gown, a lab coat, a curtain, a sheet, an automobile interior material, and a wipe.

26. A method of producing a nanoparticle of cerium oxide, comprising the following step:
Step a): a step in which an oxidizing agent is added to a solution containing condensed phosphoric acid of the following general formula (I) or/and a salt thereof and cerium (III) ion.

27. A method of producing the nanoparticle of cerium oxide according to claim 26, further comprising:
Step b): a step in which the solution obtained in the step a) is subjected to hydrothermal treatment.

28. A method of producing the nanoparticle of cerium oxide according to claim 26 or 27, wherein APHA of a 0.1 mass% dispersion of the nanoparticle of cerium oxide is 400 or less.
